# EUROPEAN PATENT SPECIFICATION

(11) **EP 1 841 878 B1**
(45) Date of publication and mention of the grant of the patent: **15.12.2010**
(21) Application number: 05799803.1
(22) Date of filing: 01.11.2005
(51) Int. Cl.: C12N 15/867, C12N 15/90, C12N 5/10, C12N 7/01, A61K 48/00

(54) **METHOD FOR THE GENERATION OF VIRUS PRODUCING CELL LINES AND CELL LINES**
VERFAHREN ZUR ERZEUGUNG VIRUSPRODUZIERENDER ZELLINIEN SOWIE ZELLINIEN
PROCEDE DE GENERATION DE LIGNEES CELLULAIRES PRODUCTRICES DE VIRUS, ET LIGNEES CELLULAIRES

(30) Priority: 02.11.2004 EP 04025919
(43) Date of publication of application: 10.10.2007
(73) Proprietor: Helmholtz-Zentrum für Infektionsforschung GmbH, 38124 Braunschweig (DE)
(72) Inventor: WIRTH, Dagmar, 38124 Braunschweig (DE); HAUSER, Hansjörg, 38302 Wolfenbüttel (DE); SCHUCHT, Roland, 38124 Braunschweig (DE)
(74) Representative: Kröncke, Rolf
(86) International application number: PCT/EP2005/011667
(87) International publication number: WO 2006/048228

(56) References cited:
- WO-A-01/25466
- WILDNER ET AL: "GENERATION OF A CONDITIONALLY neo(r)-CONTAINING RETROVIRAL PRODUCER CELL LINE: EFFECTS OF neo(r) ON RETROVIRAL TITER AND TRANSGENE EXPRESSION" GENE THERAPY, MACMILLAN PRESS LTD., BASINGSTOKE, GB, vol. 5, no. 5, 1998, pages 684-691, XP002100126 ISSN: 0969-7128
- VANIN E F ET AL: "Development of high-titer retroviral producer cell lines by using Cre-mediated recombination" JOURNAL OF VIROLOGY, THE AMERICAN SOCIETY FOR MICROBIOLOGY, US, vol. 71, no. 10, 1997, pages 7820-7826, XP002161355 ISSN: 0022-538X
- ARAI T ET AL: "A NEW SYSTEM FOR STRINGENT, HIGH-TITER VESICULAR STOMATITIS VIRUS G PROTEIN-PSEUDOTYPED RETROVIRUS VECTOR INDUCTION BY INTRODUCTION OF CRE RECOMBINASE INTO STABLE PREPACKAGING CELL LINES" JOURNAL OF VIROLOGY, THE AMERICAN SOCIETY FOR MICROBIOLOGY, US, vol. 72, no. 2, February 1998 (1998-02), pages 1115-1121, XP000857996 ISSN: 0022-538X
- FINN G K ET AL: "HOMOLOGOUS PLASMID RECOMBINATION IS ELEVATED IN IMMORTALLY TRANSFORMED CELLS" MOLECULAR AND CELLULAR BIOLOGY, vol. 9, no. 9, 1989, pages 4009-4017, XP002370446 ISSN: 0270-7306
- KARREMAN S ET AL: "ON THE USE OF DOUBLE FLP RECOGNITION TARGETS (FRTS) IN THE LTR OF RETROVIRUSES FOR THE CONSTRUCTION OF HIGH PRODUCER CELL LINES" NUCLEIC ACIDS RESEARCH, OXFORD UNIVERSITY PRESS, SURREY, GB, vol. 24, no. 9, 1 May 1996 (1996-05-01), pages 1616-1624, XP000616161 ISSN: 0305-1048
- MILLER A D ET AL: "FACTORS INVOLVED IN PRODUCTION OF HELPER VIRUS-FREE RETROVIRUS VECTORS" SOMATIC CELL AND MOLECULAR GENETICS, NEW YORK, NY, US, vol. 12, no. 2, 1 March 1986 (1986-03-01), pages 175-183, XP000563397
- LOEW R ET AL: "Simplified generation of high-titer retrovirus producer cells for clinically relevant retroviral vectors by reversible inclusion of a lox-P-flanked marker gene" MOLECULAR THERAPY, ACADEMIC PRESS, SAN DIEGO, CA,, US, vol. 9, no. 5, May 2004 (2004-05), pages 738-746, XP002312968 ISSN: 1525-0016

## Description

The present invention relates to a method for generating virus producing cell lines and master producer cell lines. In particular, the present invention relates to methods allowing the production of virus producing cell lines and master producer cell lines without conducting laborious selection work. Moreover, the present invention pertains to virus producing cell lines obtained with the methods according to the present invention as well as to virus obtained using said cell lines.

### Background of the invention

Recent successes in gene therapy clinical trials have demonstrated the high potential of the use of gene therapy to cure and slow down a vast variety of diseases. However, the efficient introduction of therapeutic genes into human cells or tissues remains the main problem to be solved.

Various methods have been suggested for introducing the therapeutic nucleotides or genes into the cells or tissue including the use of viral vectors. Generally, viral helper genes (the so-called helper functions) are expressed in cells to specifically package the therapeutic gene of interest into virus particles and to transducer the same into the target cells or tissue. Adenoviral vectors are frequently used since the particles are robust, can be easily produced in high titers and efficiently infect a broad spectrum of cells. Retroviruses, in particular the genera of mammalian C type retroviruses and lentiviruses represent the type of virus widely used for stable gene transfer and expression of the gene product. Highly sophisticated vector systems have been developed for specialised purposes. The prior art knowledge is based mainly on mammalian C type viruses. Accordingly, most gene therapy trials are based on this virus transfer system. These recombinant retroviral gene delivery methods have been extensively used in view of the following advantages: i.) the efficient entry of genetic material into cells; ii.) an active, efficient process of entry into the target cell nucleus; iii.) relatively high levels of gene expression; iv.) the potential to target particular cellular subtypes through control of the vector-target cell binding and the tissue-specific control of expression; v.) a general lack of pre-existing host immunity; and vi.) substantial knowledge and clinical experience which has been gained with such vectors.

Presently, retroviruses are successfully used for ex vivo transduction in gene therapy experiments. However, the production technology for retroviral vectors is in an early state. Therapies are hampered by i) the inherent instability of infectious viral particles, ii) the difficulty to produce large amounts of virus, and iii) the broad host range of currently available viral envelopes and the sensitivity to human complement attack. Thus for both ex vivo and in vivo applications, significant developments preclude a widespread application of retroviral gene therapy. Presently, one of the main obstacles is the production of large number of stable viruses. Said production process comprises the generation of virus, e. g. retroviral vectors, with virus producing cell lines.

Recombinant retroviruses for use in gene therapy transduce a retroviral vector encoding the effector (therapeutic) gene or therapeutic nucleotide sequence into the target cells or tissue. Presently, they are produced after transfection of the vector into so called packaging cell lines stably expressing the viral proteins gag/pol and env. This principle applies for the classical vectors based on Moloney Murine Leukaemia Viruses (MLV). Lentiviruses are mostly produced from transient expression systems since the toxicity of helper genes, in particular, of the VSV-G protein (see below) requires a timely restricted expression.

Construction of packaging cells for retroviral vectors is a tedious procedure. Sequential transfection of appropriate gag/pol and env expression constructs and time-consuming screening procedures for a balanced and high-level expression of all components are needed. Heterogeneity in expression by individual cell clones is due to i.) random integration of the helper genes into the chromosomal DNA of the host cell and ii.) variable copy numbers of the transfected constructs. Presently, subcloning and testing of individual subclones is applied to isolate suitable packaging cells.

The outer shell proteins (env) are ligands for cellular receptors mediate viral entry and define the host range of the virus. Retroviruses allow substitution of their envs and mixed retroviral particles are derived by this technique named pseudotyping. For this purpose env proteins are used which have been isolated from naturally occurring non-murine viruses (e.g. GALV, Gibbon Ape Leukaemia Virus and VSV G (Vesicular Stomatitis Virus, protein G). In addition, a large number of new env proteins have been created by recombinant techniques mainly with the objective of specifying the host range thereby achieving targeting of the virus to specific cell types, and conferring resistance to human complement. Further developments on env proteins promise improved therapies. This is also important in view of the known inherent retrovirus instability which is to a large degree due to the env proteins. The instability of the retrovirus during the production process, storage or during purification procedures is a major limitation for most applications. Presently, the only way to significantly improve retroviral stability is by replacing the env protein with the VSV G protein, resulting in particles that can be concentrated and stored for an extended time period. Despite its advantage in using VSV G for retrovirus pseudotyping, VSV G expression is toxic to producer cells. To date VSV G pseudotyped virus particles are produced in transient transfection systems or by inducible expression of the VSV G protein, thus, limiting the applicability of the system.

The use of the novel env proteins for therapeutic purposes is currently hampered by the fact that for each of the different env proteins, an individual packaging cell line has to be constructed. Each of these cell lines again has to be established according to the above described procedures since more economic methods have not yet been developed.

The above described problems apply mutatis mutandis to other viral system, like the lentivirus system, the adenovirus and the adenovirus associated virus system.

Thus, for efficient retrovirus production, viral helper genes (gag/pol and env) as well as the therapeutic vector must be expressed in a so-called "balanced" ratio in producer cell lines. According to the state of the art, packaging cell lines as the precursors are established by random integration of the viral helper genes into the host cell genome. Since the integration site dramatically influences recombinant gene expression and its stability, intensive screening is required to isolate optimal packaging cell clones. This procedure has to be followed independently for any env gene. Even once this packaging cell line is established, a second round of screening has to be performed for preparation of the therapeutic virus particles. For establishment of packaging cells producing therapeutic retroviruses, i.e. virus producing cell lines, the packaging cells have to be transfected with a therapeutic vector. The nature of the randomly chosen integration site as well as the copy number affects the resulting virus titer, again necessitating another round of time-consuming screening for efficient and stable vector expression. Obviously, this screening has to be repeated for any therapeutic virus of interest.

In particular, the laborious and time-consuming screening procedure comprises the following steps:
(i) transfection of the vector and selection of transfected single cell clones;
ii.) evaluation of the transgene expression;
iii.) evaluation of the recombinant virus titer;
iiii.) evaluation of the stability of expression and titer over longer cultivation periods;
iiv.) characterization of the chromosomal integration site and determination of vector copy numbers;
iv.) test for absence of RCRs (replication competent retroviruses).

Moreover, it was found that each virus producer cell clone needs further adaption for optimal production conditions. This work additionally covers a considerable amount of time and labour with regard to the overall production process.

To facilitate this screening in most cases the therapeutic viruses additionally carry a selection or marker gene. This marker gene allows for selecting stable integration and/or high level, long term expression. However, in the last years evidence has been arising that the transduction of additional genes can impose significant problems in therapeutical settings. One of the major problems is that an immune response against the foreign genes (e.g. selection markers) is provoked. This can result in efficient elimination of transduced cells, thereby limiting or exclude the therapeutic success. Even more concern is raised since insertional leukemogenesis was observed in the mouse model due to the transduction of a marker gene that was prior considered to be biologically inert. Thus, the overall aim to improve and to make viral gene therapy safer is to exclude any coding sequences beside the therapeutic gene of interest.

In view of the above outlined problems and obstacles a broad application of gene delivery methods using virus, in particular retrovirus, is currently not possible.

### Summary of the present invention

The present invention provides new methods for the production of virus producing cell lines. In particular, the present invention aims at providing an improved system for preparing virus that may be of subsequent use in medicine.

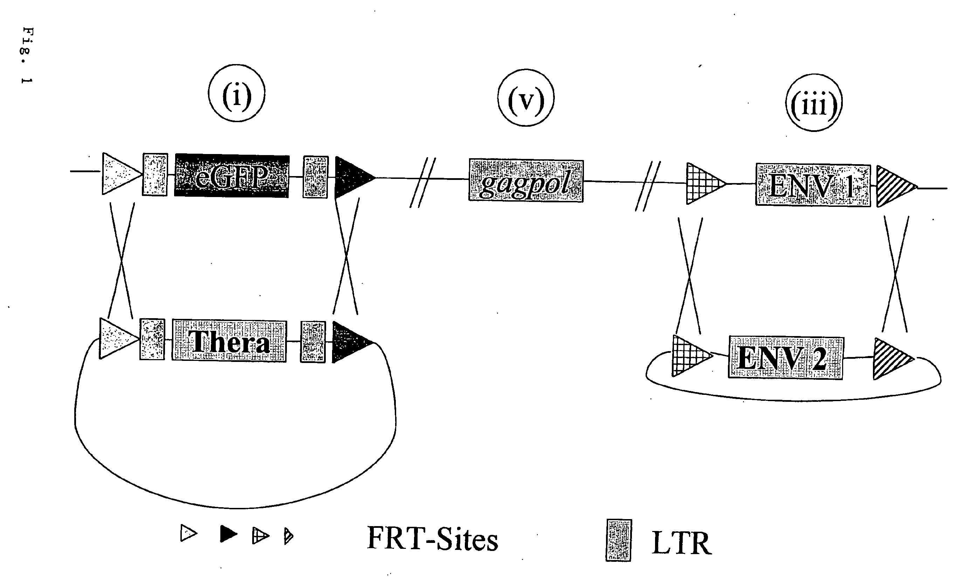

In particular, the present invention provides a method for the generation of virus producing cell lines using a master cell line once produced before. The present invention also provides methods for the production of the master producer cell line. An example of this master cell line is depicted schematically in Fig.1. Said cell line allows for easy, specific and predictable integration of expression cassettes containing the nucleotides or gene of interest (Fig.1 (i)) and/or the envelope moieties (Fig.1 (iii)) necessary to produce the desired type of virus. Due to the predicted integration site, the titer, the cultivation conditions and the safety record is not affected.

When using the master cell line no additional laborious screening of the cells is necessary after targeting the expression cassette of interest into the already tagged chromosomal loci in the master cell line. Thus, allowing simple exchange of various expression cassettes while maintaining the properties of the master cell line which was once selected for high expression chromosomal integration site allowing high and predictable virus production under known culture conditions.

The present invention further relates to the virus, producing cell line obtainable with the method according to the present invention. The virus producing cell line according to the present invention allow for standardised production of virus which may be eventually used in gene therapy. In addition, the present invention relates to the master producer cell line obtainable with the method according to the present invention. Finally, the present invention concerns the use of said cells for the production of virus

### Brief description of the figures

### Figure 1:

Master producer cell line with tagged integration sites for exchange of retroviral vector and env gene.

This overview exemplifies the outline of the generation of therapeutic virus producer cell lines. In a first step, a retroviral tagging vector and the helper functions gagpol and env are stably integrated into the host's genome. Both the retroviral vector and the env gene are flanked by FRT sites, thereby allowing for site-specific replacement of the expression cassettes by a therapeutic vector and/or an alternative env gene.

### Figure 2:

### Generation of a master producer cell line with one exchangeable cassette

A: A retroviral tagging vector was established that carries a reporter gene (GFP), an EMCV IRES element and a hygromycin resistance gene fused to the thymidine kinase gene (EMCV element is not shown for better overview). In the 3' U3 region of the LTR a tagging cassette was integrated comprising two non-interacting FRT-sites (shown as triangles) and a start codon deficient, thus, non-functional neomycin phosphotransferase (npt) gene (upper lane). This vector was stably transduced into PG13 packaging cells. For tagging, 293 cells were infected with the virus supernatant using a multiplicity of infection below 0,01 to ensure single copy integration. In infected cells, the retroviral vector is stably integrated according to the lower lane. As a consequence of reverse transcription, the 3'U3 region including the tagging cassette is duplicated to the 5' end, resulting in the depicted proviral state.
B: Infected, tagged cells were analyzed according to their GFP expression. The figure shows an overlay histogram of a flow cytometry analysis of a tagged cell clone (thick line) compared to non-infected cells (thin line).
C: The tagged cell clone 1B2 was transfected in subsequent steps with the viral helper functions, i.e. the gagpol expression vector pCeB (Cosset et al., J. Virol. 69:7430-7436, 1995) and pENVA-his (Spitzer et al., J. Virol 77, 6070-6075, 2003), an amphotropic env expression vector. Cells were selected for stable integration. Cells were screened for balanced expression of helper function gagpol and env, resulting in a high retroviral titer. The figure shows the efficiency of release of GFP transducing retroviruses of individual subclones (1B2-8-F, IIIC2, and A-E) as compared to the tagged cell line 1 B2.
D: The virus production of clone 1 B2-8F over the time is shown. As demonstrated virus production is stable over 12 weeks.

### Figure 3:

### Generation of a therapeutic virus producing cell line

A: The master cell clone 1B2-8-F was targeted according to the depicted scheme. A targeting vector was constructed in which the therapeutic CollagenVII transducing retrovirus vector was flanked with the non-interacting FRT sites and further carries an ATG codon and an additional IRES element to initiate neo expression. Upon targeting npt expression is obtained via IRES mediated translation initiation from an LTR derived RNA.
B. G418 resistant cell clones obtained upon targeting of the retroviral vector cassette were analyzed for GFP expression. The histogram overlay shows the GFP expression of G418 resistant clones in comparison to the parental 1B2-8-F clone and 293 cells as a control.
C: Correct targeting was confirmed by Southern blotting of G418 resistant clones. For this purpose, chromosomal DNA of parental master cell line 1B2-8-F and IIIC2 and independent subclones was digested with Sacl and hybridyzed against an EMCV probe. Correct targeting results in a 2 kb fragment in correctly targeted clones and elimination of the 4 kb parental signal. Such analysis was conducted for about 50 independently raised subclones, over 95% of them showing correct targeted exchange (not shown).
D: Targeted cells were analyzed according to the expression of ColVII. For this purpose cells were immunostained using an antibody against hColVII. As a control the tagged cells were stained.

### Figure 4:

A: Virus production was monitored. Mouse NIH3T3 cells were infected with the virus supernatant of a G418 resistant subclone. Infected and control cells were immunostained for hColVII.
B: The ColVII virus titer was monitored after targeting the vector pMSIRcolVII into 1B2-8-F cells. G418 resistant clones were isolated, targeted exchange was confirmed by Southern blotting (not shown) and analyzed for their virus production. The figure B shows the uniform titer of subclones generated upon transfection according to the protocol described in the example.
C: The retroviral titer of a single G418 resistant targeted subclone was monitored over time. Fig. C shows the stability of the titer over 4 weeks.

### Figure 5:

### Exchange of the tagging cassette GFP with different cassettes

Shown are the titer of the parental cell line, M, and the titer of pMSIReGFP targeted clones, lanes 1 to 8, and of pMLIReGFP targeted clones, lanes a to e. High and reproducible virus titer are obtained in independent subclones after targeting the tagged GFP cassette by Flp recombination.

### Table 1:

### Efficiency of targeted exchange and retroviral titers upon targeting

On the left side, the various constructs used for the targeting experiments described in example 3 are shown.

On the right side, the targeting efficiency with the various vectors is illustrated. Highly efficient targeting is achieved. Furthermore, the obtained clones show correct targeting with very high average titer.

### Detailed description of the invention

It is noted that as used herein the following terms have the meaning as indicated below:
The term "nucleotide of interest, NOI" as used herein is intended to mean a nucleic acid molecule which can include eukaryotic mRNA, cDNA from eukaryotic mRNA, genomic sequences from eukaryotic DNA and synthetic DNA or RNA sequences. Preferably the NOI is a nucleic acid sequence derived from a human DNA or RNA sequence. For example, the NOI encodes a molecule selected from the group consisting of proteins, peptides, antisense nucleic acid sequences, ribozymes and siRNA.

The term "operably linked" as used herein is intended to mean arrangements of elements wherein the components so described are configured so as to perform their usual function. Thus, a given promotor operably linked to a coding sequence is capable of effecting the expression of the coding sequence when the proper enzyme is present.

The term "virus" is interchangeably used with the term "virus particle" in the present application.

The term "virus producing cell (line)" as used herein is intended to mean a cell which contains all elements necessary for the production of virus or virus particle, including all cis elements necessary for virus production.

The term "cell line" refers to a population of isogenic cells capable of continuous growth and division in vitro.

The term "master producer cell (line)" as used herein is intended to mean a cell or cell line which contain chromosomally integrated at least a sequence encoding gag/pol, an expression cassette containing sequences allowing site specific integration, sequence(s) for a selection marker and a non-functional selection domain, and a different expression cassette that preferably but does not necessarily contain sequences allowing site specific integration, sequence(s) for a selection marker different to the selection marker in the other expression cassette and a non-functional selection domain.

The term "selection marker" as used herein is intended to mean a marker which enables discrimination of moieties, like cells, which may or may not contain said selection marker. For example, a selection marker may be the expression of a specific molecule, a resistance marker, like a marker conferring drug-resistance to a cell, etc. Thus, the selection marker allows for simpler preparation, manufacturing, characterization or testing of the cell or cell line.

The term "functional selection domain" as used herein is intended to mean a domain being functional in that it contains an element allowing the selection of cells. The functional selection domain comprises at least two non-functional selection fragments.

Non-functional selection fragments means that the selection element is not present in a functional form and, hence, can not be used for selecting purposes between different cells. "Non-functional selection fragments" are fragments which do not contain all elements necessary to allow the selection based on the selection element. The combination of at least two complementing non-functional selection fragments results in a functional selection domain. The selection domain may be a selection marker as indicated above, like a resistance marker or another marker that allows for characterization of the cells. Further, the non-functional selection fragment may be a silent but functional selection marker. That is, the functional selection marker is not transcribed due to a missing promoter or missing enhancer or missing translation initiator sequences. It is noted that the selection marker of the selection domain should be different to the selection marker of the expression cassette present in the master producer cell line.

The term "site-specific integration" as used herein is intended to mean that integration of elements, like expression cassettes, is performed at predefined sites only.

The term "tagged" as used herein is intended to mean a state in which a chromosomal site is genetically marked/labelled by integration of a nucleotide sequence that allows for site specific integration and subsequent selection.

The term "targeted" as used herein is intended to mean the state a nucleotide sequence has been site specifically integrated into a previously tagged chromosomal integration site.

The present invention relates to a method for the generation of a virus producing cell line comprising the following steps:
a.) introducing into a master producer cell line a nucleotide construct comprising a nucleotide of interest (NOI) cassette comprising:
   i) at least one NOI operably linked to a promoter;
   ii) long terminal repeats (LTR) located upstream and downstream of the NOI sequence, respectively;
   iii) a non-functional selection fragment Ia located downstream of the 3' LTR allowing the formation of a functional selection domain I when combined with the complementary fragment Ib present in the master producer cell line; and
   iv) sequences allowing site-specific integration of the NOI cassette into a first expression cassette present in said master producer cell line, said sequences flank the NOI cassette on both sites of said cassette; and
b.) introducing into the master cell producer cell line a nucleotide construct comprising an env expression cassette comprising
   i) sequence(s) of env gene(s) operably linked to a promoter;
   ii) a non-functional selection fragment IIa allowing the formation of a functional selection domain II which may be identical or different from the functional selection domain I when combined with the complementary fragment IIb present in the master producer cell line, said non-functional selection fragment IIa being located downstream of the sequence(s) of env gene(s);
   iii) sequences allowing site-specific integration of said env-cassette into a second tagged expression cassette present in said master producer cell line, said sequences flank the env cassette on both sites of said cassette;
c.) initiating replacement of the NOI cassette and/or env cassette with the corresponding expression cassette present in the master producer cell line;
d.) selecting cell lines having stably integrated the NOI cassette and/or the env cassette on the basis of the functional selection domain(s);
wherein the order of step a.) and b.) is not determined and wherein steps c.) and d.) may be conducted after each of steps a.) and b.) or after conducting steps a.) and b.);
with the proviso that the master producer cell line used in a.) and b.), respectively, is obtainable by
I) stably integrating an expression cassette into the genome of a host cell or cell line, said expression cassette comprises a nucleotide construct comprising from the 5' and to the 3' end:
   i) a first sequence allowing for site-specific integration;
   ii) a promoter;
   iii) a sequence encoding a selection marker A, said sequence is operably linked with the promoter of ii);
   iv) a second sequence allowing for site-specific integration, said sequence is different from the first sequence of i);
   v) a non-functional selection fragment Ib;
II) selecting a transduced cell line wherein the expression cassette of (I) is stably integrated into the host genome resulting in a transduced cell line tagged with the expression cassette of I);
III) stably integrating a different expression cassette into the genome of the cell or cell line, said expression cassette comprises a nucleotide construct comprising from the 5' end to the 3' end:
   i) a first sequence allowing for site-specific integration;
   ii) a promoter;
   iii) a sequence encoding a selection marker B which is different from selection marker A, said sequence is operably linked with the promoter of ii);
   iv) a second sequence allowing for site-specific integration, said sequence is different from the first sequence of i);
   v) a non-functional selection fragment IIb which may be the same or may be different to the selection fragment Ib;
   wherein the sequences allowing site-specific integration may be identical or may be different to said sequences of the expression cassette of I);
IV) selecting a transduced cell line wherein the expression cassette of (III) is stably integrated into the host genome resulting in a transduced cell line tagged with the expression cassette of (III);
V) stably integrating a different expression cassette into the genome of the cell or cell line, said expression cassette comprises a nucleotide construct containing a promoter operably linked to a sequence encoding gag/pol;
VI) selecting a transduced cell line wherein the expression cassette of (V) is stably integrated into the host genome resulting in a transduced cell line tagged with the expression cassette of V;
and wherein the order of the integration of the different expression cassettes according to (I), (III) and (V) is not determined.

In another preferred embodiment, the present invention relates to a method for the generation of a virus producing cell line comprising the following steps:
a.) introducing into a master producer cell line a nucleotide construct comprising a nucleotide of interest (NOI) cassette comprising:
   i) at least one NOI operably linked to a promoter;
   ii) long terminal repeats (LTR) located upstream and downstream of the NOI sequence, respectively;
   iii) a non-functional selection fragment Ia located downstream of the 3' LTR allowing the formation of a functional selection domain I when combined with the complementary fragment Ib present in the master producer cell line; and
   iv) sequences allowing site-specific integration of the NOI cassette into a first expression cassette present in said master producer cell line, said sequences flank the NOI cassette on both sites of said cassette; and optionally
b.) introducing into the master cell producer cell line a nucleotide construct comprising an env expression cassette comprising
   i) sequence(s) of env gene(s) operably linked to a promoter;
   ii) a non-functional selection fragment IIa allowing the formation of a functional selection domain II which may be identical or different from the functional selection domain I when combined with the complementary fragment IIb present in the master producer cell line, said non-functional selection fragment IIa being located downstream of the sequence(s) of env gene(s);
   iii) sequences allowing site-specific integration of said env-cassette into an env expression cassette present in said master producer cell line, said sequences flank the env cassette on both sites of said cassette;
c.) initiating replacement of the NOI cassette and/or env cassette with the corresponding expression cassette present in the master producer cell line;
d.) selecting cell lines having stably integrated the NOI cassette and/or the env cassette on the basis of the functional selection domain(s);
   wherein the order of step a.) and optional b.) is not determined and wherein step c.) and d.) may be conducted after each of steps a.) and b.) or after conducting steps a.) and b.);
   with the proviso that the master producer cell line used in a.) and b.), respectively, is obtainable by

I) stably integrating an expression cassette into the genome of a host cell or cell line, said expression cassette comprises a nucleotide construct comprising from the 5' and to the 3' end:
   i) a first sequence allowing for site-specific integration;
   ii) a promoter;
   iii) a sequence encoding a selection marker A, said sequence is operably linked with the promoter of ii);
   iv) a second sequence allowing for site-specific integration, said sequence is different from the first sequence of i);
   v) a non-functional selection fragment Ib;
II) selecting a transduced cell line wherein the expression cassette of (I) is stably integrated into the host genome resulting in a transduced cell line tagged with the expression cassette of I);
III) stably integrating an env expression cassette into the genome of said cell or cell line, said env expression cassette comprises a nucleotide construct comprising:
   i) optionally a first sequence allowing for site-specific integration;
   ii) a promoter;
   iii) sequence(s) of env gene(s) operably linked to the promoter of ii);
   iv) optionally a second sequence allowing for site-specific integration, said sequence is different from the first sequence of i);
   v) optionally a non-functional selection fragment IIb which may be the same or may be different to the selection fragment Ib;
   wherein the sequences allowing site-specific integration may be identical or may be different to said sequences of the expression cassette of I);
(IV) selecting a transduced cell line wherein the expression cassette of (III) is stably integrated into the host genome resulting in a transduced cell line tagged with the expression cassette of (III);
(V) stably integrating a different expression cassette into the genome of the cell or cell line, said expression cassette comprises a nucleotide construct containing a promoter operably linked to a sequence encoding gag/pol;
(VI) selecting a transduced cell line wherein the expression cassette of (V) is stably integrated into the host genome resulting in a transduced cell line tagged with the expression cassette of V;
and wherein the order of the integration of the different expression cassettes according to (I), (III) and (V) is not determined.

In a first step, a master producer cell line is produced according to steps (I) to (VI) of the above methods. Of course, when starting the method with a cell or cell line containing already one of the elements to be introduced into the cell, only the remaining steps have to be performed.

In addition, the integration of the different cassettes according to (I), (III) and (V) may be conducted at the same time. In particular, the integration of the gag/pol and another expression cassette, in particular the env expression cassette may be performed simultaneously.

With the first stage a "master producer cell line" is produced. This master producer cell line is generated once providing in a first embodiment of the present invention a tagged cell line having incorporated into its genome a gag/pol expression cassette and two expression cassettes each containing a selection marker. In a second embodiment, the method according to the present invention provides a tagged cell line having incorporated into its genome a gag/pol expression cassette, an env expression cassette which is optionally substitutable and a further expression cassette containing a replaceable selection marker.

Thus, the master producer cell line represents a cell line selected for optimized expression of the various expression cassettes. The selection steps have to be performed only once for each expression cassette tagged into the cell line.

In the second stage according to the method of the present invention, the master cell line is targeted with the NOI and the env gene of interest if the master producer cell line contains two expression cassettes containing two different selection markers. In another embodiment the master cell line is targeted with the NOI and optionally an env gene of interest if the master producer cell line contains an expression cassette containing a selection marker and a second expression cassette containing an env gene.

That is, the second stage comprises a gene cassette replacement wherein the replacement of the gene cassette takes place at the previously tagged sites, the sequences of site specific integration, in the genome of the master producer cell line. Thus, the cassette(s) are integrated into the tagged chromosomal loci of the master producer cell line. The integration of the cassette(s) of interest into the particular known sites results in virus producing cell lines having an optimal expression of the targeted cassettes. In addition, since the replacement takes place at the sites that allow site specific integration in the host genome, no laborious selection steps are necessary. In particular, the introduced expression cassette(s) will not integrate randomly into the host genome but specifically into the tagged chromosomal loci having the site specific integration sites. Consequently, in the final virus producer cell line the chromosomal sites into which the helper components and the retroviral vector are integrated are identical to the corresponding sites of the master producer cell line. Thus, the final virus producer cell line will have exactly the same properties as the master producer cell line, in particular in view of the efficiency of virus production, fermentation conditions and safety features. Further, the rate of correctly targeting the cassette is 95% to 100 %, preferably 97% to 100%. That means almost all clones obtained after the targeting step contain a correctly targeted replacement cassette.

Moreover, the presence of the LTR sequences in the NOI cassette allows for the production of a recombinant viral genome which does not contain any additional sequences, e.g. sequences encoding a selection marker, like resistance to antibiotics, etc. The NOI cassette and also the env cassette do not contain any gene coding for a selection marker within the LTR but the fragments of the selection domains preset in the cassettes are outside the LTR sequences, thus, said selection marker sequences will not be present in the virus. Further, the virus does not contain additional sequences derived from the replacement step, like sequences allowing site-specific integration, e.g. a loxP site or a FRT site. Hence, the virus or virus particle produced according to the method of the present invention is free of additional components which may adversely affect the therapeutical benefits of the NOI. It is within the scope of the present invention that the LTR sequences may be substituted with sequences having the same function, i.e. enabling the generation of viral vector containing the NOI.

In more detail, the first stage resulting in a master producer cell line comprises the steps of tagging chromosomal integration sites by integrating the different expression cassettes into the host genome and selecting appropriate cells. At least one of the different expression cassettes introduced into the genome comprise sequences at the 5' -site of the cassette allowing for site-specific integration, further, an additional promoter is present downstream of the above mentioned 5'-site. The additional promotor is operably linked with a sequence encoding a selection marker A and B, respectively. Selection markers A and B are different from each other. The at least one cassette having a site-specific integration site at the 5' end also contains at the 3' -site a second sequence allowing for site-specific integration. Further downstream a non-functional selection fragment Ib and Ib, respectively, is present. The site specific integration sites at the 5' end and the 3' end are different from each other.

With "sequences allowing site specific integration" sequences are meant which allow for a replacement exchange of the cassette integrated into the host genome, the tagged cassette, with a different cassette, the targeting cassette, which should become integrated into the genome by replacement of the integrated tagged cassette. Preferably, a recombinase assisted mechanism is used for the replacement exchange. This system facilitates the exchange. Out of the known recombinase systems the Cre recombinase/ loxP recognition sites of bacteriophage P1 or the site-specific FLP recombinase of S. cerevisiae being the most frequently used.

The general method conducted in the first stage to establish a transduced cell line or master producer cell line containing three tagged cassettes, i.e. the gag/pol cassette, an env cassette and a third expression cassette containing the NOI is well known in the art. The selection of the known master producer cell lines is based on various expression characteristics like the expression of a selection marker, introduced within the tagging cassette. Additional criteria for the selection of an appropriate cell line are an optimal expression level that is stable over time, the maintenance of fermentation conditions, the cell growth, the genomic stability of the integration site and safety features. However, the specific integration of the expression cassette containing the selection marker in combination with the non-functional selection domain and the sequences for site-specific integration are not known in the art.

The order of the integration of the expression cassettes according to steps (I), (III) and (V) is not determined. That means, one may start with the integration of the gag/pol cassette first. Moreover, if cell lines are already present having at least one of the cassettes of steps (I), (III) and (V) stably integrated in their genome, said cell lines may be used for further integration of the cassettes not yet integrated in the genome. Further, at least two cassettes may be integrated simultaneously. In particular, it is possible to introduce the gag/pol and another expression cassette, in particular, the env cassette, at the same time for integration into the cell genome.

The expression cassette to be integrated in steps (I) and/or (III) comprises at least the following elements:
Site-specific integration elements at the 5' and 3' end of the selection marker sequence. These elements include recognition sites for enzymes which enable specific exchange for sequences flanked by the same recognition sites into the specific chromosomally integrated nucleotide sequence. A typical example of this system is the yeast Flp recombinase system. To avoid the deletion of the integrated product at a later time point, it is preferred to use two non-interacting recombination sites at the 5' end and 3' end.

A promoter and/or another element (e.g. an IRES element) for enabling transcription and/or translation of the selection marker.

A selection marker. The selection marker may be a drug-resistance marker like neomycin phosphotransferase, an expression marker within the cell (e.g. GFP, β-galactosidase) or at its surface (e.g. receptors) or it may be an env protein itself.

A non-functional selection fragment. This non-functional selection fragment represents an incomplete selection marker, e.g. a drug resistance marker. Since the fragment represents an incomplete domain, selection on the basis of this specific selection marker is not possible, but it requires completion with at least one additional non-functional selection fragment. This additional fragment is supplied later by the integration of the NOI and/or env cassette according to steps a) and/or b) respectively, thus, only specific and precise integration in step c) of the claimed process render the cell in a position to be selectable on the basis of the selection marker present in the selection domain.

As indicated above, the sequences allowing site specific integration at the 5' and 3' site of the cassettes are preferably non-interacting. Using different sequences avoids unwanted excision of the insert. Moreover, the sequences for site specific integration may be identical or may be different in the cassettes of step (I) and (III). Preferably, said sequences are different from each other. Thus, specific integration or exchange of only one cassette is conducted.

The selection process for selecting transduced cells having chromosomally integrated the desired cassette is conducted according to well known techniques taking the desired characteristic of the transduced cells into account, like high-level production, stability and safety.

The first stage of the method according to the present invention results in a transduced cell line or master producer cell line having chromosomally integrated usually at least three cassettes. The integration sites are also referred to as the tagged chromosomal loci. This cell line is selected for the desired parameters, like stability of the integrated cassettes, safety, high-level production if applicable, etc. When using the env protein as one of the selection markers A or B, the resulting cells have all genetic elements required for recombinant virus production. In addition, if a sequence encoding the env protein is used for the generation of the master cell line, wherein the sequence encoding the env protein represents the sequence encoding either selection marker A or B, the tagged env cassette does not necessarily contain sequences at the 5' and 3' end allowing site-specific integration.

In the second stage the virus producing cell line is established. By using site-specific integration sites being identical to the site specific integration sites present in the chromosomally integrated cassettes, it is possible to specifically replace the cassette present in the master producer cell line with the NOI and, optionally, env cassette.

The use of the master producer cell line allows for a site specific integration efficiency of more than 95%. In addition, the expression characteristics are maintained after the exchange of the tagged selection marker introduced in the first stage with the NOI and/or env cassette. Moreover, different clones obtained in the second stage of the method according to the present invention demonstrate a titer that is stable and predictable in all subclones.

The NOI cassette or the env cassette introduced in the second stage of the method according to the present invention comprises the site-specific integration sites at the 5' and 3' side flanking the cassettes. These site specific integration sites are identical to at least one of the site specific integration sites present in the expression cassettes which are chromosomally integrated into the master producer cell line. Moreover, the cassettes introduced during the second stage contain a non-functional selection fragment which allows the formation of a functional selection domain when combined with the complementary fragment present in the tagged chromosomal loci.

The formation of a functional selection domain provides for an extremely effective selection procedure for correct cassette replacement. Thus, laborious selection work is avoided but only cells wherein the correct cassette replacement took place can be selected on the basis of the functional selection domain.

The functional selection domain contains a selection marker, like a drug-resistance selection marker, antibiotics etc. For example, the tagged chromosomal locus of the master producer cell line encompasses the sequence for encoding drug-resistance except for the initiation codon or the promotor sequence. By supplementing the fragment with the non-functional selection fragment present in the targeting cassette (NOI and/or env cassette), the missing in frame initiation codon or the promoter is introduced, thus, providing a functional selection domain which can be used for selection purposes.

The above described system overcomes the laborious and time-consuming work for selection suitable clones. Moreover, the method according to the present invention enables the use of the master producer cell line for the production of a variety of different virus producing cell lines for the production of virus having different env proteins or containing viral vector encoding different nucleotides of interest, e.g. different therapeutic genes etc. allowing the expression of therapeutically useful proteins in the target cell.

Moreover, simple and fast pseudotyping of the virus is possible. The term pseudotyping means incorporating in at least a part of, or substituting at least a part of, or replacing all of, an env gene of a viral genome with a heterologous env gene, for example an env gene from another virus. Pseudotyping can improve viral vector stability and transduction efficiency and specificity.

If necessary, the cassettes introduced into the cell or cell line contain a promotor which is operably linked with the selection marker or the NOi or the env sequence. The promotor may be selected from known promotor sequences including constitutive promoters (e.g. CMV, and promoters for timely restricted (controlled) expression (e.g. Tet-controllable, tTA dependent promoters).

However, an additional promotor besides the LTR sequences is not necessary, since the LTR itself represents a structure promoting the transcription of the sequences downstream of the LTR sequence, probably be readthrough transcription.

Thus, the NOI cassette additionally comprises all genetic retroviral cis-elements required for proper packaging of the RNA, reverse transcription and integration, e.g. flanking long terminal repeat (LTR). The LTRs are responsible for proviral integration, and transcription. They also serve as enhancer-promoter sequences. That means, the LTRs can control the expression of the recombinant therapeutic genes. Flanking of the NOI sequence by the LTRs enables the production of viral vectors which do not contain any additional sequence encoding a molecule which may adversely affect the therapeutic effectiveness of the virus. In particular, the viral vector does not contain any sequence encoding for a selection marker or other molecules necessary during the production process of the virus. The retroviral cis-elements include the retroviral long terminal repeats, the primer binding site, the packaging signal and the polypurine tract (PPT).

In a preferred embodiment, the promoter to which the NOI and/or the env gene is operably linked, is the LTR sequence located upstream of the NOI and env, respectively. In another preferred embodiment, e.g. when preparing a SIN vector, an additional promoter sequence is present in the NOI and env cassette, respectively, e.g. one of the promoters mentioned above.

In a further preferred embodiment, the viral vector produced by the virus producing cell line is a self inactivating retroviral vector (SIN). SIN vectors have a modified U3 region of the 3' LTR wherein the transcriptional enhancers are deleted leading to a non-functional LTR promoter after reverse transcription and integration of the viral cassette into the host genome. Thus, in a preferred embodiment of the present invention the NOI cassette to be integrated according to the present invention has a modified 3'LTR sequence, a so called SIN LTR.

In a preferred method according to the present invention, the expression cassettes of steps (I) and (III) are transduced by retroviral gene transfer and therefore contain 5' LTR and 3' LTR. In the U3 region of the 3' LTR the non-functional selection fragment may be integrated. Further, the 3' LTR contains site-specific integration sites. As a consequence of the process of retroviral infection the insert of the 3'U3 insert is duplicated to the U3 of the 5'LTR, thereby flanking the expression cassettes with site-specific integration sites.

Alternatively, the introduction of the cassettes to be integrated into the cells is performed by non-retroviral transfection methods which are well known to the skilled artisan. For example, the introduction of the cassettes may comprise infection with viral vectors, transfection with liposomes also called lipofection, transfection using the well known calciumphosphate technique or electroporation. Transfection generally refers to the uptake of foreign DNA by a cell.

The replacement of the expression cassettes integrated into the master producer cell line according to steps (I) and (III) is conducted for example with the help of a recombinase or a restriction enzyme. That is, the enzyme necessary to promote or catalyse the exchange is for example is the Cre recombinase or the FLP recombinase or any other recombinase known in the art. Alternatively, the exchange may be promoted by using specific restriction enzymes like SCEI.

The genes, encoding the above mentioned enzymes may be introduced into the cell by transfection or infection of appropriate expression vectors. The transfection may be a stable or a transient transfection. Moreover, the recombinase system may be an inducible system. Alternatively, the enzyme as such may be directly provided into the cell culture system.

The selection of cell lines having stably integrated the NOI-cassette and/or the env cassette is conducted on the basis of the expression of the selection markers present in the selection domain. Due to the replacement of the cassettes both non-functional selection domain fragments are assembled to result in a functional selection domain. In particular, the supply of one of the necessary non-functional selection fragments to complete the non-functional selection fragment in the tagged chromosomal locus provides for a selection marker, e.g. drug-resistance marker gene having functional activity only in cells where an exchange of cassettes took place. Thus, the selection is an easy and reliable process overcoming the laborious and time consuming work which is necessary to select suitable clones.

Further, since the process maintains and does not affect the structure of the chromosomal integration sites, the expression of the NOI will have the same expression characteristics as the marker gene in the master producer cell line.

The present invention further relates to the virus producing cell line itself. The virus producing cell according to the present invention affords the production of virus or virus particle containing a viral vector. Importantly, this vector does not need to contain any coding sequence which may adversely affect the therapeutic benefits of the NOI. By providing 5' LTR and 3' LTR upstream and downstream of the NOI sequence, respectively, together with the known cis acting elements, psi and the polypurine tract required for packaging and reverse transcription, it is possible to obtain a viral vector containing only the NOI sequence. This viral vector is packaged into the virus which may subsequently be used to infect the target cell. In a particular preferred embodiment the viral vector is a self inactivating retroviral vector.

Within the scope of the present invention, there is the virus obtainable with the claimed method. The virus is characterized in having a viral vector which does not contain any additional sequence which product may adversely affect the therapeutic benefits of the NOI present in the viral vector. In particular, the virus according to the present invention does not need to encode for a selection marker being used during the production process for the generation of the virus.

Thus, the virus according to the present invention is particularly useful in gene therapy. Hence, the present invention relates also to methods for treating patients using the virus according to the present invention. Moreover, the present invention relates to a therapeutic or pharmaceutical preparation for use in gene therapy. The skilled person is well aware of the composition containing the virus as an active ingredient.

Preferably, the virus is a retrovirus, for example mouse retroviruses or lentiviruses. In particular retrovirus include: mammalian C-type viruses like Moloney murine leukaemia virus (Mo-MLV), Moloney sarcoma virus (Mo-MSV), Abelson murine leukaemia virus (A-MLV), mammalian B-type viruses like mouse mammary tumour virus (MMTV), avian leukosis-sarcoma virus like Rous sarcoma virus (RSV) and Fujinami sarcoma virus (FuSV), lentiviruses like human immunodeficiency virus (HIV) and simian immunoddeficiency virus (SIV), equine infectious anaemia virus (EIAV), Spumavirus and human T-cell leukemia virus HTLV.

The env introduced into the master producer cell line to arrive at a virus producing cell line according to the present invention, may be the env obtained from the same virus as the gag pol proteins. Alternatively an env protein from a heterologous virus may be used, when pseudotyping the virus. Pseudotyping may improve viral stability and transduction efficiency, specificity of transduction as explained above. The env proteins to be used in the method according to the present invention may include env 10A1, 4070A, ecotropic env, envs from GALV, RD114 and LMCV, and genetically modified envs e.g. that confer stability to complement, or target specific cell types, and chimeric envs.

In addition, master producer cell lines producing toxic or harmful proteins like VSV-G can be established using expression cassettes that provide a timely restricted (conditional) expression of the protein.

In the method according to the present invention the host cell used for the production of the master producer cell line may be selected from the group consisting of mammalian cells like HT1080, HEK293, Tert-immortalized cell lines etc. The skilled person is well aware of suitable cell or cell lines usable for the production of the master producer cell line and the generation of the virus producing cell line according to the present invention.

The present invention also provides the use of a viral vector, in particular of a retroviral vector, of the invention in the manufacture of a pharmaceutical composition The pharmaceutical composition may be used to deliver a NOI to a target cell in need of the same. In particular, the pharmaceutical composition may be used in gene therapy. The composition may optionally contain a pharmaceutically acceptable carrier, diluent, excipient or adjuvant. The composition may additionally contain known pharmaceutical ingredients like binder, etc.

### Examples

### Example 1 Generation of a master producer cell line

### Step a Tagging cells with a GFP reporter cassette

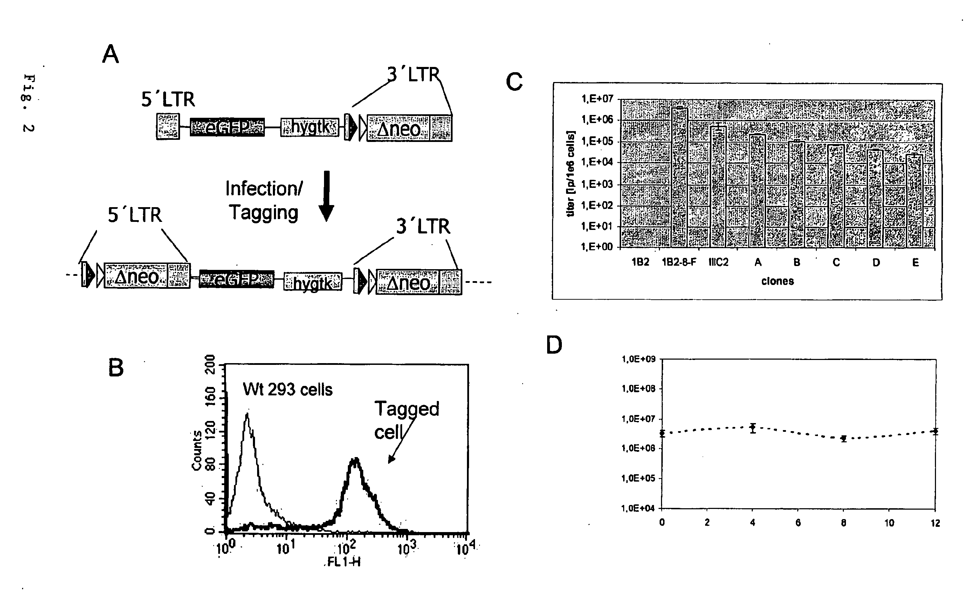

A retroviral tagging vector pROL was constructed. This vector contains MSCV derived LTRs and transduces a bicistronic expression cassette comprising GFP (green fluorescent protein) and hygromycin B phosphotransferase. In addition, in the 3'LTR (Nhel site) two non-interacting FRT sites and a sequence comprising an ATG-deficient, non-functional neomycin phosphotransferase gene was integrated (see Fig.2a, upper line for a schematic drawing of the vector). This vector (pROL) was stably transfected into the PG13 packaging cell line as described in Spitzer et al, Hum Gene Ther. 10, 1893-1902 (1999). Briefly, 5µg of pROL was transfected to 1x10⁵ cells. The cells were selected for Hygromycin resistance (200 U/ml). The GFP titer was determined. Subsequently, 1x10⁵ HEK293 cells were infected in the presence of 8µg/ml polybrene as described in Spitzer et al, Hum Gene Ther. 10, 1893-1902 (1999), thereby applying a multiplicity of infection below 0,01. Two days after infection, cells were selected for Hygromycin resistance. Hygromycin resistant cells with optimal GFP expression were isolated by Fluorescence activated cell sorting (FACS) (Fig. 2b).

### Step b integration of env and gag/pol

GFP high-expressing clones were checked for single copy integration by Southern blot analysis. Subsequently, single clones were sequentially cottransfected with the helper functions. For this purpose, 5 µg of the amphotropic env expression vector pENVA-his (Spitzer et al., J. virol 77, 6070-6075 (2003)) was transfected to 3x10⁵ cells of the tagged cell clone. Cells were selected in medium containing 11 mM Histidinol and env expression was confirmed on isolated clones by flow cytometry. Then, pCeB, a gag/pol expression vector conferring resistance to Blasticidin (Cosset, et al J. Virol. 69:7430-7436 (1995)), was transfected, again using 5µg of vector and 3x10⁵ cells. Cell clones resistant to 3µg/ml Blasticidin were further characterized. Clones were screened for maximal production of GFP transducing retroviruses (Fig. 2c). Single cell clones were monitored stable retrovirus production. Fig. 2d shows virus production from clone 1 B2-8F. In this example, the second expression construct (envA) is not exchangeable.

### Example 2 Generation of a virus producer cell line

### Exchange of the tagging cassette GFP with a collagen expression cassette

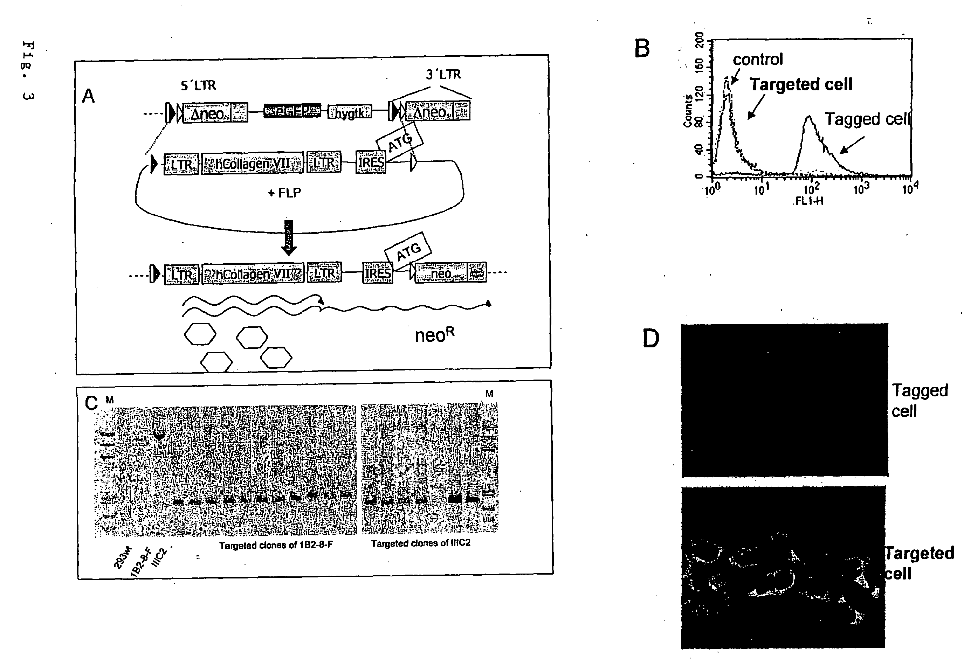

A therapeutic vector was integrated according to Fig. 3a. 2 µg of the targeting vector pMSIRcolVII (containing an MLV based retroviral vector transducing human ColVII cDNA) was cotransfected with 10µg of a Flp recombinase encoding vector pSVFIpe using DNA/Calcium phosphate coprecipitation on 3x10⁵ HEK293 cells on 6 well plates as described in Spitzer et al, Hum Gene Ther. 10, 1893-1902 (1999). The medium was refreshed 6 hours post transfection. The cells were incubated for 4 days to allow the targeting process take place, and then 1*10⁴ to 5*10⁵ cells were seeded on 10 cm culture plates and set under selection pressure of G418 (1000µg/ml) for at least two weeks. Single cell clones were isolated and correct targeting was confirmed by loss of GFP expression (Fig.3b) and Southern Blot analysis (Fig.3c). Immunostaining against ColVII showed a signal specifically in targeted cells (Fig 3d).

### Stable titer of cells with correctly targeted cells

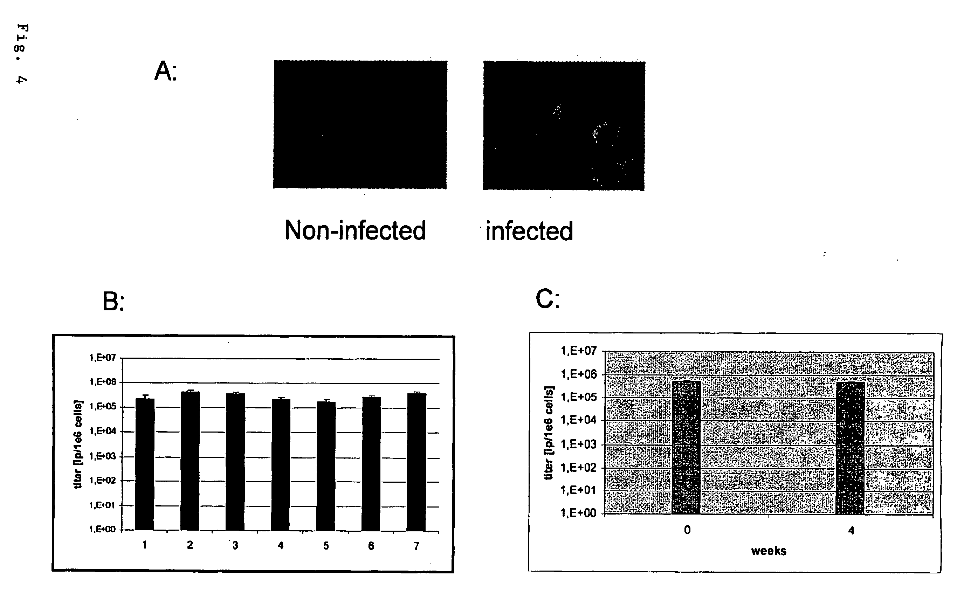

The virus supernatant of ColVII targeted cells was used to infect NIH3T3 cells in presence of 8µg/ml polybrene (Spitzer et al, Hum Gene Ther. 10, 1893-1902 (1999)). Expression of ColVII was documented by immunostaining using a ColVII antibody (Fig. 4a). Virus production after targeting was analyzed. Fig 4b shows the virus titer of independent cell clones obtained upon targeting and selection for G418 resistance. Importantly, all analyzed targeted subclones show a consistant (Fig 4b) and stable (Fig 4c) titer for at least 4 weeks proving that this method leads to predictable and stable virus production.

### Example 3 Optimization of retroviral vector design

### Exchange of the tagging cassette GFP with alternative GFP cassettes

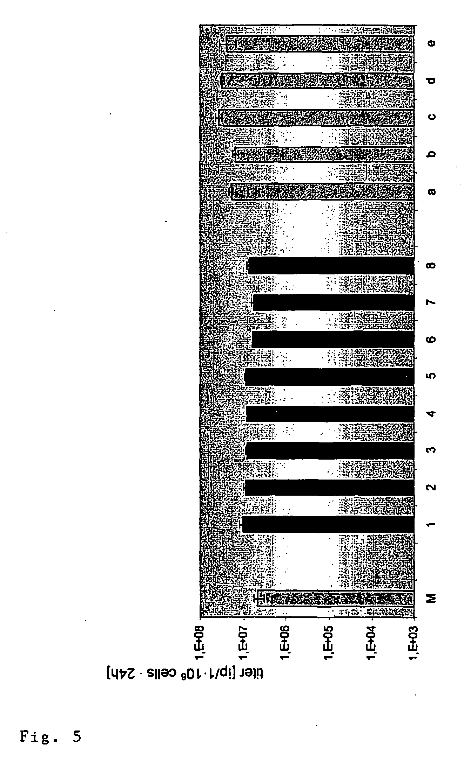

To evaluate the efficiency of virus production different GFP vectors were integrated according to Fig. 3a. The vectors are summarised in Table 1. In this experiment, both the MSCV and MLV derived LTRs were included as well as a self inactivating vector with an internal elFa promoter. 2 µg of the targeting vectors pMSIReGFP, pMLIREGFP and pSINeGFP (Table1), respectively, were cotransfected with 10µg of a Flp recombinase encoding vector pSVFIpe using DNA/Calcium phosphate coprecipitation on 3x10⁵ HEK293 cells on 6 well plates as described in Spitzer et al, Hum Gene Ther. 10, 1893-1902 (1999). The medium was refreshed 6 hours post transfection. The cells were incubated for 4 days to allow the targeting process take place, and then 1*10⁴ to 5*10⁵ cells were seeded on 10 cm culture plates and set under selection pressure of G418 (1000µg/ml) for at least two weeks. Single cell clones were isolated and correct targeting was confirmed by Southern Blot analysis (not shown). The titer was determined (Table 1). Fig. 5A shows the titer of the parental cell line (M) as well as the titer of pMSIReGFP targeted (lanes 1 to 8) and pMLIReGFP targeted (lanes a to e) clones.

The result of the targeting experiments is summarized in Table 1. It shows that targeting is highly efficient, and high titers are obtained from differently designed targeting vectors including self-inactivating vectors.

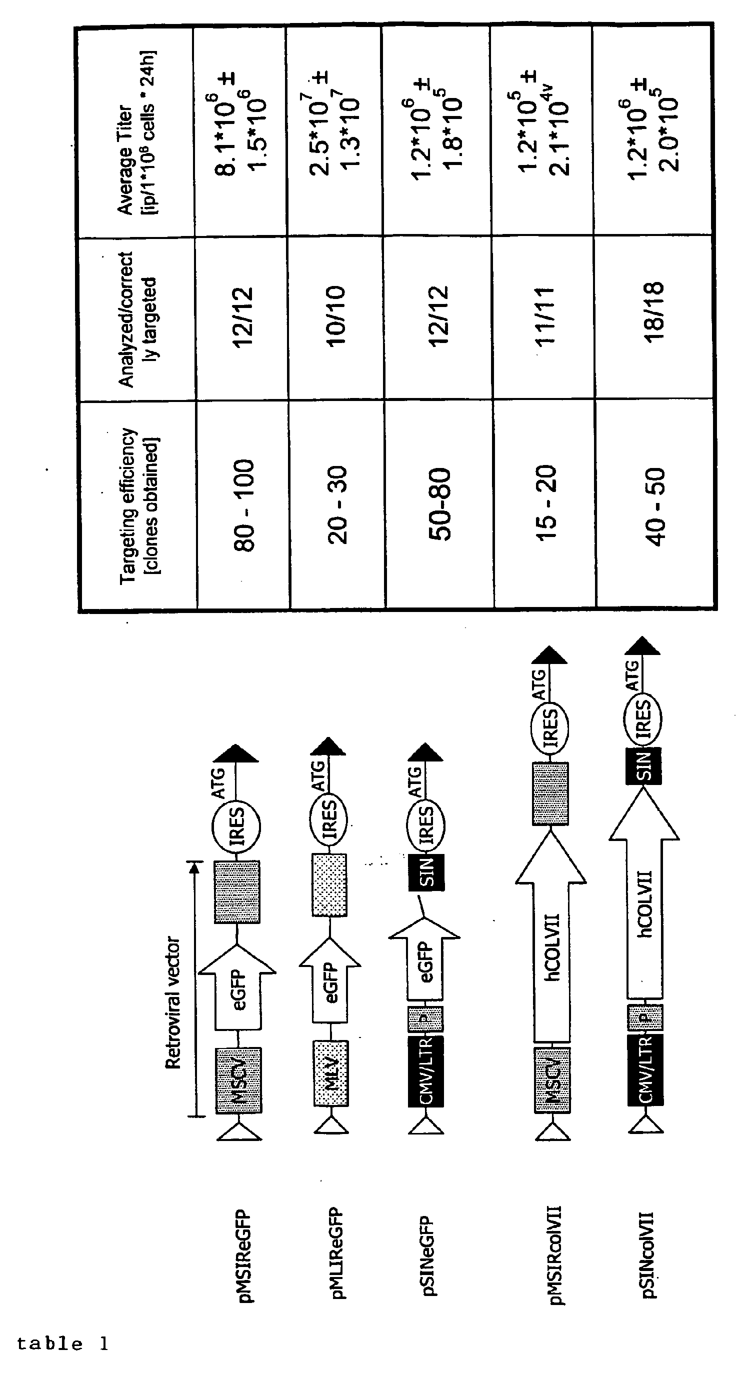

## Claims

1. Method for the generation of a virus producing cell line comprising the following steps:
a.) introducing into a master producer cell line a nucleotide construct comprising a nucleotide of interest (NOI) cassette comprising:
i) at least one NOI operably linked to a promoter;
ii) long terminal repeats (LTR) located upstream and downstream of the NOI sequence, respectively;
iii) a non-functional selection marker fragment Ia located downstream of the 3' LTR allowing the formation of a functional selection marker I when combined with the complementary marker fragment Ib present in the master producer cell line; and
iv) sequences allowing site-specific integration of the NOI cassette into a first expression cassette present in said master producer cell line, said sequences flank the NOI cassette on both sites of said cassette; and
b.) introducing into the master cell producer cell line a nucleotide construct comprising an env expression cassette comprising
i) sequence(s) of env gene(s) operably linked to a promoter;
ii) a non-functional selection marker fragment IIa allowing the formation of a functional selection marker II which may be identical or different from the functional selection marker I when combined with the complementary marker fragment IIb present in the master producer cell line, said non-functional selection marker fragment IIa being located downstream of the sequence(s) of env gene(s);
iii) sequences allowing site-specific integration of said env-cassette into a second tagged expression cassette present in said master producer cell line, said sequences flank the env cassette on both sites of said cassette;
c.) initiating replacement of the NOI cassette and/or env cassette with the corresponding expression cassette present in the master producer cell line;
d.)selecting cell lines having stably integrated the NOI cassette and/or the env cassette on the basis of the functional selection marker(s);
wherein the order of step a.) and b.) is not determined and wherein steps c.) and d.) may be conducted after each of steps a.) and b.) or after conducting steps a.) and b.);
with the proviso that the master producer cell line used in a.) and b.), respectively, is obtainable by
I) stably integrating an expression cassette into the genome of a host cell or cell line, said expression cassette comprises a nucleotide construct comprising from the 5' and to the 3' end:
i) a first sequence allowing for site-specific integration;
ii) a promoter;
iii) a sequence encoding a selection marker A, said sequence is operably linked with the promoter of ii);
iv) a second sequence allowing for site-specific integration, said sequence is different from the first sequence of i);
v) a non-functional selection marker fragment Ib allowing the formation of a functional selection marker I when combined with the complementary fragment Ia present in the NOI cassette;
II) selecting a transduced cell line wherein the expression cassette of (I) is stably integrated into the host genome resulting in a transduced cell line tagged with the expression cassette of I);
III) stably integrating a different expression cassette into the genome of the cell or cell line, said expression cassette comprises a nucleotide construct comprising from the 5' end to the 3' end:
i) a first sequence allowing for site-specific integration;
ii) a promoter;
iii) a sequence encoding a selection marker B which is different from selection marker A, said sequence is operably linked with the promoter of ii);
iv) a second sequence allowing for site-specific integration, said sequence is different from the first sequence of i);
v) a non-functional selection marker fragment IIb allowing the formation of a functional selection marker II when combined with the complementary fragment IIa present in the env expression cassette which may be the same or may be different to the selection marker fragment Ib;
wherein the sequences allowing site-specific integration may be identical or may be different to said sequences of the expression cassette of I);
IV) selecting a transduced cell line wherein the expression cassette of (III) is stably integrated into the host genome resulting in a transduced cell line tagged with the expression cassette of (III);
V) stably integrating a different expression cassette into the genome of the cell or cell line, said expression cassette comprises a nucleotide construct containing a promoter operably linked to a sequence encoding gag/pol;
VI) selecting a transduced cell line wherein the expression cassette of (V) is stably integrated into the host genome resulting in a transduced cell line tagged with the expression cassette of V;
and wherein the order of the integration of the different expression cassettes according to (I), (III) and (V) is not determined and wherein the selection marker I and II are different to A or B.

2. Method for the generation of a virus producing cell line comprising the following steps:
a.) introducing into a master producer cell line a nucleotide construct comprising a nucleotide of interest (NOI) cassette comprising:
i) at least one NOI operably linked to a promoter;
ii) long terminal repeats (LTR) located upstream and downstream of the NOI sequence, respectively;
iii) a non-functional selection fragment Ia located downstream of the 3' LTR allowing the formation of a functional selection domain I when combined with the complementary fragment Ib present in the master producer cell line; and
iv) sequences allowing site-specific integration of the NOI cassette into a first expression cassette present in said master producer cell line, said sequences flank the NOI cassette on both sites of said cassette; and optionally
b.) introducing into the master cell producer cell line a nucleotide construct comprising an env expression cassette comprising
i) sequence(s) of env gene(s) operably linked to a promoter;
ii) a non-functional selection marker fragment IIa allowing the formation of a functional selection marker II which may be identical or different from the functional selection marker I when combined with the complementary marker fragment IIb present in the master producer cell line, said non-functional selection marker fragment IIa being located downstream of the sequence(s) of env gene(s);
iii) sequences allowing site-specific integration of said env-cassette into an env expression cassette present in said master producer cell line, said sequences flank the env cassette on both sites of said cassette;
c.) initiating replacement of the NOI cassette and/or env cassette with the corresponding expression cassette present in the master producer cell line;
d.) selecting cell lines having stably integrated the NOI cassette and/or the env cassette on the basis of the functional selection domain(s);
wherein the order of step a.) and optional b.) is not determined and wherein step c.) and d.) may be conducted after each of steps a.) and b.) or after conducting steps a.) and b.);
with the proviso that the master producer cell line used in a.) and b.), respectively, is obtainable by
I) stably integrating an expression cassette into the genome of a host cell or cell line, said expression cassette comprises a nucleotide construct comprising from the 5' and to the 3' end:
i) a first sequence allowing for site-specific integration;
ii) a promoter;
iii) a sequence encoding a selection marker A, said sequence is operably linked with the promoter of ii);
iv) a second sequence allowing for site-specific integration, said sequence is different from the first sequence of i);
v) a non-functional selection marker fragment Ib allowing the formation of a functional selection marker I when combined with the complementary fragment Ia present in the NOI cassette;
II) selecting a transduced cell line wherein the expression cassette of (I) is stably integrated into the host genome resulting in a transduced cell line tagged with the expression cassette of I);
III) stably integrating an env expression cassette into the genome of said cell or cell line, said env expression cassette comprises a nucleotide construct comprising:
i) optionally a first sequence allowing for site-specific integration;
ii) a promoter;
iii) sequence(s) of env gene(s) operably linked to the promoter of ii);
iv) optionally a second sequence allowing for site-specific integration, said sequence is different from the first sequence of i);
v) optionally a non-functional selection marker fragment IIb allowing the formation of a functional selection marker II when combined with the complementary fragment IIa present in the env expression cassette which may be the same or may be different to the selection marker fragment Ib;
wherein the sequences allowing site-specific integration may be identical or may be different to said sequences of the expression cassette of I);
(IV) selecting a transduced cell line wherein the expression cassette of (III) is stably integrated into the host genome resulting in a transduced cell line tagged with the expression cassette of (III);
(V) stably integrating a different expression cassette into the genome of the cell or cell line, said expression cassette comprises a nucleotide construct containing a promoter operably linked to a sequence encoding gag/pol;
(VI) selecting a transduced cell line wherein the expression cassette of (V) is stably integrated into the host genome resulting in a transduced cell line tagged with the expression cassette of V;
and wherein the order of the integration of the different expression cassettes according to (I), (III) and (V) is not determined and wherein the selection marker I and II are different to selection marker A or B.

3. A method for the generation of a master producer cell line comprising the steps of
I) stably integrating an expression cassette into the genome of a host cell or cell line, said expression cassette comprises a nucleotide construct comprising from the 5' and to the 3' end:
i) a first sequence allowing for site-specific integration;
ii) a promoter;
iii) a sequence encoding a selection marker A, said sequence is operably linked with the promoter of ii);
iv) a second sequence allowing for site-specific integration, said sequence is different from the first sequence of i);
v) a non-functional selection marker fragment Ib allowing the formation of a functional selection marker I when combined with the complementary fragment Ia present in the NOI cassette;
II) selecting a transduced cell line wherein the expression cassette of (I) is stably integrated into the host genome resulting in a transduced cell line tagged with the expression cassette of I);
III) stably integrating a different expression cassette into the genome of the cell or cell line, said expression cassette comprises a nucleotide construct comprising from the 5' end to the 3' end:
i) a first sequence allowing for site-specific integration;
ii) a promoter;
iii) a sequence encoding a selection marker B which is different from selection marker A, said sequence is operably linked with the promoter of ii);
iv) a second sequence allowing for site-specific integration, said sequence is different from the first sequence of i);
v) a non-functional selection marker fragment IIb allowing the formation of a functional selection marker II when combined with the complementary fragment IIa present in the env expression cassette which may be the same or may be different to the selection marker fragment Ib;
wherein the sequences allowing site-specific integration may be identical or may be different to said sequences of the expression cassette of I);
IV) selecting a transduced cell line wherein the expression cassette of (III) is stably integrated into the host genome resulting in a transduced cell line tagged with the expression cassette of (III);
V) stably integrating a different expression cassette into the genome of the cell or cell line, said expression cassette comprises a nucleotide construct containing a promoter operably linked to a sequence encoding gag/pol;
VI) selecting a transduced cell line wherein the expression cassette of (V) is stably integrated into the host genome resulting in a transduced cell line tagged with the expression cassette of V;
and wherein the order of the integration of the different expression cassettes according to (I), (III) and (V) is not determined.

4. A method for the generation of a master producer cell line comprising the steps of:
I) stably integrating an expression cassette into the genome of a host cell or cell line, said expression cassette comprises a nucleotide construct comprising from the 5' and to the 3' end:
i) a first sequence allowing for site-specific integration;
ii) a promoter;
iii) a sequence encoding a selection marker A, said sequence is operably linked with the promoter of ii);
iv) a second sequence allowing for site-specific integration, said sequence is different from the first sequence of i);
v) a non-functional selection marker fragment Ib allowing the formation of a functional selection marker I when combined with the complementary fragment Ia present in the NOI cassette;
II) selecting a transduced cell line wherein the expression cassette of (I) is stably integrated into the host genome resulting in a transduced cell line tagged with the expression cassette of I);
III) stably integrating an env expression cassette into the genome of said cell or cell line, said env expression cassette comprises a nucleotide construct comprising:
i) optionally a first sequence allowing for site-specific integration;
ii) a promoter;
iii) sequence(s) of env gene(s) operably linked to the promoter of ii);
iv) optionally a second sequence allowing for site-specific integration, said sequence is different from the first sequence of i);
v) optionally a non-functional selection marker fragment IIb allowing the formation of a functional selection marker II when combined with the complementary fragment IIa present in the env expression cassette which
may be the same or may be different to the selection marker fragment Ib;
wherein the sequences allowing site-specific integration may be identical or may be different to said sequences of the expression cassette of I);
(IV) selecting a transduced cell line wherein the expression cassette of (III) is stably integrated into the host genome resulting in a transduced cell line tagged with the expression cassette of (III);
(V) stably integrating a different expression cassette into the genome of the cell or cell line, said expression cassette comprises a nucleotide construct containing a promoter operably linked to a sequence encoding gag/pol;
(VI) selecting a transduced cell line wherein the expression cassette of (V) is stably integrated into the host genome resulting in a transduced cell line tagged with the expression cassette of V;
and wherein the order of the integration of the different expression cassettes according to (I), (III) and (V) is not determined.

5. The method according to any one of claims 1 to 4 wherein the expression cassettes of (I) and/or (III) additionally contain a 5' and 3' LTR.

6. The method according to any one of claims 1 to 5 wherein the introduction of the nucleotide construct(s) is performed by transfection or an infection.

7. The method according to any one of claims 1, 2,5, 6 wherein the integration of the cassettes is effected by a recombinase system and/or by restriction enzymes.

8. The method according to claim 7 wherein the recombinase system is the Cre recombinase / loxP recognition sites of bacteriophage P1 or the site-specific FLP recombinase of S. cerevisiae.

9. The method according to claim 1 or 2 wherein the recombination system for site-specific integration is the FLP system using different FLP recognition target (FRT) variants wherein the sequences flanking the NOI-cassette and the env-cassette on both sites and which allow site-specific recombination are non-interacting sequences being different from each other.

10. The method according to any one of claims 1, 2, 5 to 9 wherein the non-fuctional selection marker fragment present in the NOI-cassette and/or the env-cassette contains a promoter or an IRES element operably linked to an initiation codon-sequence allowing the expression of a selection marker different to the selection marker A or B when complemented with the complementary non-functional selection marker fragment.

11. The method according to any one of claims 1, 2, 5 to 10 wherein the non-functional selection marker fragment present in the NOI-cassette and/or the env-cassette contains only a promoter that activates a silent but functional selection marker integrated upon the tagging step.

12. The method according to any one of claims 1, 2, 5 to 11 wherein the non-functional selection marker fragment present in the NOI-cassette and/or the env-cassette contains a splice signal that allows for translation of the selection marker.

13. The method according to claim 1 or 2 wherein the NOI encodes a molecule selected from the group consisting of proteins, peptides, antisense nucleic acid sequences, ribozymes, and siRNA.

14. The method according to claim 1, 2 or 13 wherein the NOI is a therapeutically useful molecule.

15. The method according to any one of claims 1, 3, 5 to 14 wherein the selection markers A and/or B are selected from the group consisting of antibiotics, drug-resistance, cellular and extracellular expression markers.

16. The method according to claim 15 wherein the selection markers A and/or B are selected from the group consisting of GFP, β-galactosidase, secreted alkaline phosphatase, and cell surface markers.

17. The method according to any one of claims 1,2, 5 to 16 wherein the promoter to which the NOI and/or the env gene is operably linked according to a.) i) or b.) i) is a LTR located upstream of the NOI and/or env gene, respectively.

18. The method according to any one of the preceding claims wherein at least one cassette contains an additional promoter operably linked to the sequences to be transcribed.

19. The method according to claim 18 wherein the additional promoter is selected from the group consisting of constitutive promoters and promoters for timely restricted or controlled expression.

20. The method according to any one of claims 1, 2, 5 to 19 wherein the recombinase is introduced in the packaging cell by transfection or infection.

21. The method according to any one of claims 1, 2, 5 to 19 wherein the recombinase is introduced into the system by directly providing the enzyme to the cells.

22. The method according to any one of claims 1, 2, 5 to 19 wherein the gene encoding the recombinase is stably integrated into the host genome operably linked with an inducible promotor.

23. The method according to any one of the preceding claims wherein the virus is pseudotyped.

24. Virus producing cell line obtainable with a method according to any one of claims 1, 2, and 5 to 23.

25. Master producer cell line obtainable with a method according to any one of claims 3 to 22.

26. Use of the virus producing cell line according to claim 24 or the master producer cell line according to claim 25 for the production of virus.

27. Use according to claim 26 for the production of a therapeutic preparation containing a virus or virus particle for gene therapy.

## Patentansprüche

1. Verfahren zur Generierung einer Virus-produzierenden Zelllinie umfassend die folgenden Schritte:
a) Einbringen eines Nukleotidkonstrukts umfassend eine Kassette mit dem interessierenden Nukleotid (nucleotide of interest, NOI) umfassend:
i) mindestens ein NOI, das mit einem Promoter operativ verknüpft ist;
ii) (lange terminale Wiederholungen, long terminal repeats, LTR) die sich stromaufwärts beziehungsweise stromabwärts der NOI Sequenz befinden;
iii) ein nicht-funktionales Selektionsmarker-Fragment Ia, das sich stromabwärts vom 3' LTR befindet und die Ausbildung eines funktionalen Selektionsmarkers I ermöglicht, wenn es mit dem komplementären Marker-Fragment Ib, das sich in der Master-Producer Zelllinie befindet, kombiniert wird; und
iv) Sequenzen, die eine ortsspezifische Integration der NOI Kassette in eine erste Expressionskassette, die sich in dieser Master-Producer Zelllinie befindet, erlaubt, diese Sequenzen flankieren die NOI Kassette an beiden Seiten dieser Kassette und
b.) Einbringen eines Nukleotidkonstrukts enthaltend eine Kassette für die env-Expression in die Master-Producer Zelllinie umfassend
i) Sequenz(en) des/der env-Gen(e), die operativ mit einem Promoter verbunden sind;
ii) ein nicht-funktionales Selektionsmarker-Fragment IIa, das die Ausbildung eines funktionalen Selektionsmarkers II erlaubt, wobei dieser gleich unter verschieden sein kann zu dem funktionalen Selektionsmarker I, wenn dieses Fragment mit dem komplementären Markerfragment IIb, das in der Master-Producer Zelllinie vorhanden ist, kombiniert wird, dieses nicht-funktionale Selektionsmarker-Fragment IIa befindet sich stromabwärts der Sequenz(en) des/der env-Gen(e);
iii) Sequenzen, die eine ortsspezifische Integration der env-Kassette in eine zweite getaggte Expressionskassette, die in der Master-Producer Zelllinie vorhanden ist, erlaubt, diese Sequenzen flankieren die env-Kassette an beiden Seiten dieser Kassette;
c) initiieren des Austausches der entsprechenden Expressionskassette aus der Master-Producer Zelllinie mit der NOI Kassette und/oder env-Kassette;
d) Auswählen von Zelllinien die stabil die NOI Kassette und/oder die env-Kassette integriert haben auf Basis des/der funktionalen Selektionsmarkers; wobei die Reihenfolge von Schritt a) und b) nicht bestimmt ist und wobei die Schritte c) und d) nach jedem Schritt der Schritte a) und b) durchgeführt werden kann oder nach Durchführung von Schritten a) und b);
unter der Voraussetzung, dass die Master-Producer Zelllinie wie sie in a) und b) verwendet wird, erhältlich ist durch
I) stabiles Integrieren einer Expressionskassette in das Genom einer Wirtszelle oder Zelllinie, diese Expressionskassette umfasst ein Nukleotidkonstrukt umfassend vom 5' zum 3' Ende:
i) eine erste Sequenz, die eine ortsspezifische Integration erlaubt;
ii) ein Promoter;
iii) eine Sequenz, die ein Selektionsmarker A kodiert, diese Sequenz ist operativ mit dem Promoter von ii) verknüpft;
iv) eine zweite Sequenz, die die ortsspezifische Integration ermöglicht, diese Sequenz unterscheidet sich von der ersten Sequenz i);
v) ein nicht-funktionales Selektionsmarker-Fragment Ib, das die Ausbildung eines funktionalen Selektionsmarkers I erlaubt, wenn es mit dem komplementären Fragment Ia, wie es in der NOI Kassette vorhanden ist, kombiniert wird;
II) Auswahl einer transduzierten Zelllinie worin die Expressionskassette I stabil in das Wirtsgenom integriert ist resultierend in eine transduzierte Zelllinie getaggt mit der Expressionskassette I;
III) stabiles Integrieren einer anderen Expressionskassette in das Genom der Zell oder Zelllinie, diese Expressionskassette umfasst ein Nukleotidkonstrukt umfassend von 5' zum 3' Ende:
i) eine erste Sequenz, die die ortsspezifische Integration erlaubt;
ii) ein Promoter
iii) eine Sequenz kodierend für einen Selektionsmarker B, der sich von dem Selektionsmarker A unterscheidet, diese Sequenz ist operativ mit dem Promoter von ii) verknüpft;
iv) eine zweite Sequenz, die die ortsspezifische Integration erlaubt, diese Sequenz unterscheidet sich von der ersten Sequenz i);
v) ein nicht-funktionales Selektionsmarker-Fragment IIb, das die Ausbildung eines funktionalen Selektionsmarkers II erlaubt, wenn mit dem komplementären Fragment IIa, das sich in der env Expressionskassette befindet, kombiniert wird, dieses kann gleich oder verschieden sein zu dem Selektionsmarkerfragment Ib;
wobei die Sequenzen die eine ortsspezifische Integration erlauben, gleich oder verschieden zu den Sequenzen der Expressionskassette I sein können;
IV) Auswahl einer transduzierten Zelllinie, in die die Expressionskassette III stabil in das Wirtsgenom integriert vorliegt, resultierend in eine transduzierte Zelllinie getaggt mit der Expressionskassette von III;
V) stabiles Integrieren einer weiteren Expressionskassette in das Genom der Zelle oder Zelllinie, diese Expressionskassette umfasst ein Nukleotidkonstrukt enthaltend ein Promoter, der operativ mit einer Sequenz kodierend gag/pol verknüpft ist, enthält;
VI) Auswahl einer transduzierten Zelllinie, wobei die Expressionskassette von V) stabil in das Wirtsgenom integriert ist resultierend in einer transduzierte Zelllinie getaggt mit der Expressionskassette von V); und wobei die Reihenfolge der Integration der verschiedenen Expressionskassetten nach I, III und V nicht bestimmt ist und wobei die Selektionsmarker i) und ii) unterschiedlich sind zu A oder B.

2. Verfahren zur Generierung einer Virus-produzierenden Zelllinie umfassend die folgenden Schritte:
a) Einbringen eines Nukleotidkonstrukts umfassend ein interessierendes Nukleotid (nucleotide of interest, NOI) Kassette in die Master-Producer Zelllinie umfassend:
i) mindestens ein NOI operativ mit einem Promoter verknüpft;
ii) lange terminale Wiederholungen, long terminal repeats, (LTR); die sich stromaufwärts und stromabwärts der NOI Sequenz befinden;
iii) ein nicht funktionales-Selektionsfragment Ia, das sich stromabwärts des 3' LTR befindet, dies erlaubt die Ausbildung einer funktionalen Selektionsdomäne I, wenn sie mit dem komplementären Fragment Ib, das sich in der Master-Producer Zelllinie befindet, kombiniert wird; und
iv) Sequenzen, die eine ortsspezifische Integration der NOI Kassette in eine erste Expressionskassette, die in der Master-Producer Zelllinie vorhanden ist, erlaubt, diese Sequenzen flankieren die NOI Kassette an beiden Seiten der Kassette; und gegebenenfalls
b.) Einbringen eines Nukleotidkonstrukts in die Master-Producer Zelllinie, dieses umfasst eine env Expressionskassette umfassend:
i) Sequenz(en) des/der env-Gen(e), die operativ mit einem Promoter verbunden sind;
ii) ein nicht-funktionales Selektionsmarkerfragment IIa, das die Ausbildung eines funktionalen Selektionsmarkers II erlaubt, wobei dieser gleich unter verschieden sein kann zu dem funktionalen Selektionsmarker I, wenn dieses Fragment mit dem komplementären Markerfragment IIb, das in der Master-Producer Zelllinie vorhanden ist, kombiniert wird, dieses nicht funktionale Selektionsmarkerfragment IIa befindet sich stromabwärts der Sequenz(en) des/der env-Gen(e);
iii) Sequenzen, die eine ortsspezifische Integration der env-Kassette in eine in der Master-Producer Zelllinie vorliegenden env-Kassette erlaubt, diese Sequenzen flankieren die env-Kassette an beiden Seiten dieser Kassette;
c) initiieren des Austausches der entsprechenden Expressionskassette aus der Master-Producer Zelllinie mit der NOI Kassette und/oder env-Kassette;
d) Auswählen von Zelllinien die stabil die NOI Kassette und/oder die env-Kassette integriert haben auf Basis des/der funktionalisierten Selektionsmarkers; wobei die Reihenfolge von Schritt a) und b) nicht bestimmt ist und wobei die Schritte c) und d) nach jedem Schritt der Schritte a) und b) durchgeführt werden kann oder nach Durchführung von Schritten a) und b);
unter der Voraussetzung, dass die Master-Producer Zelllinie wie sie in a) und b) verwendet wird, erhältlich ist durch
I) stabiles Integrieren einer Expressionskassette in das Genom einer Wirtszelle oder Zelllinie, diese Expressionskassette umfasst ein Nukleotidkonstrukt umfassend vom 5' zum 3' Ende:
i) eine erste Sequenz, die eine ortsspezifische Integration erlaubt;
ii) ein Promoter;
iii) eine Sequenz, die ein Selektionsmarker A kodiert, diese Sequenz ist operativ mit dem Promoter von ii) verknüpft;
iv) eine zweite Sequenz, die die ortsspezifische Integration ermöglicht, diese Sequenz unterscheidet sich von der ersten Sequenz i);
v) ein nicht-funktionales Selektionsmarker-Fragment Ib, das die Ausbildung eines funktionalen Selektionsmarker I erlaubt wird, wenn es mit dem komplementären Fragment Ia, wie es in der NOI Kassette vorhanden ist, kombiniert wird;
II) Auswahl einer transduzierten Zelllinie worin die Expressionskassette I stabil in das Wirtsgenom integriert ist resultierend in eine transduzierte Zelllinie getaggt mit der Expressionskassette I;
III) stabiles Integrieren einer env Expressionskassette in das Genom dieser Zelle oder Zelllinie, die env Expressionskassette umfasst ein Nukleotidkonstrukt umfassend:
i) gegebenenfalls eine erste Sequenz, die eine ortsspezifische Integration erlaubt;
ii) einen Promoter;
iii) Sequenz(en) für env Gen(e), die operativ mit dem Promoter von ii) verknüpft sind;
iv) gegebenenfalls eine zweite Sequenz, die eine ortsspezifische Integration erlaubt, diese Sequenz unterscheidet sich von der ersten Sequenz aus i);
v) gegebenenfalls ein nicht-funktionale Selektionsmarker-Fragment IIb, das die Ausbildung eines funktionalen Selektionsmarkers II erlaubt, wenn es mit dem komplementären Fragment IIa, das sich in der env Expressionskassette befindet, kombiniert wird, dieses Fragment kann gleich oder verschieden sein zu dem Selektionsmarkerfragment Ib; wobei die Sequenzen, die die ortsspezifische Integration erlauben, gleich oder verschieden sein können zu den Sequenzen in der Expressionskassette nach I;
IV) Auswahl einer transduzierten Zelllinie, in die die Expressionskassette III stabil in das Wirtsgenom integriert vorliegt, resultierend in eine transduzierte Zelllinie getaggt mit der Expressionskassette von III;
V) stabiles Integrieren einer weiteren Expressionskassette in das Genom der Zelle oder Zelllinie, diese Expressionskassette umfasst ein Nukleotidkonstrukt enthaltend ein Promoter, der operativ mit einer Sequenz kodierend gag/pol verknüpft ist, enthält;
VI) Auswahl einer transduzierten Zelllinie, wobei die Expressionskassette von V) stabil in das Wirtsgenom integriert ist resultierend in einer transduzierte Zelllinie getaggt mit der Expressionskassette von V); und wobei die Reihenfolge der Integration der verschiedenen Expressionskassetten nach I, III und V nicht bestimmt ist und wobei die Selektionsmarker i) und ii) unterschiedlich sind zu A oder B.

3. Verfahren zur Generierung einer Master-Producer Zelllinie umfassend die Schritte:
I) stabiles Integrieren einer Expressionskassette in das Genom einer Wirtszelle oder Zelllinie, diese Expressionskassette umfasst ein Nukleotidkonstrukt umfassend vom 5' zum 3' Ende:
i) eine erste Sequenz, die eine ortsspezifische Integration erlaubt;
ii) ein Promoter;
iii) eine Sequenz, die ein Selektionsmarker A kodiert, diese Sequenz ist operativ mit dem Promoter von ii) verknüpft;
iv) eine zweite Sequenz, die die ortsspezifische Integration ermöglicht, diese Sequenz unterscheidet sich von der ersten Sequenz i);
v) ein nicht-funktionales Selektionsmarker-Fragment Ib, das die Ausbildung eines funktionalen Selektionsmarker I erlaubt wird, wenn es mit dem komplementären Fragment Ia, wie es in der NOI Kassette vorhanden ist, kombiniert wird;
II) Auswahl einer transduzierten Zelllinie worin die Expressionskassette I stabil in das Wirtsgenom integriert ist resultierend in einer transduzierten Zelllinie getaggt mit der Expressionskassette I;
III) stabiles Integrieren einer anderen Expressionskassette in das Genom der Zell oder Zelllinie, diese Expressionskassette umfasst ein Nukleotidkonstrukt umfassend von 5' bis zum 3':
i) eine erste Sequenz, die die autospezifische Integration erlaubt;
ii) ein Promoter
iii) eine Sequenz kodierend für einen Selektionsmarker B, der sich von dem Selektionsmarker A unterscheidet, diese Sequenz ist operativ mit dem Promoter von ii) verknüpft;
iv) eine zweite Sequenz, die die ortsspezifische Integration erlaubt, diese Sequenz unterscheidet sich von der ersten Sequenz i);
v) ein nicht-funktionales Selektionsmarker-Fragment IIb, das die Ausbildung eines funktionalen Selektionsmarkers II erlaubt, wenn mit dem komplementären Fragment IIa, das sich in der env Expressionskassette befindet, kombiniert wird, dieses kann gleich oder verschieden sein zu dem Selektionsmarker-Fragment Ib;
wobei die Sequenzen die eine ortsspezifische Integration erlauben, gleich oder verschieden zu den Sequenzen der Expressionskassette I.) sein können;
IV) Auswahl einer transduzierten Zelllinie, in die die Expressionskassette III stabil in das Wirtsgenom integriert ist, resultierend in eine transduzierte Zelllinie getaggt mit der Expressionskassette von III.);
V) stabiles Integrieren einer weiteren Expressionskassette in das Genom der Zelle oder Zelllinie, diese Expressionskassette umfasst ein Nukleotidkonstrukt enthaltend ein Promoter, der operativ mit einer Sequenz kodierend gag/pol verknüpft ist, enthält;
VI) Auswahl einer transduzierten Zelllinie, wobei die Expressionskassette von V) stabil in das Wirtsgenom integriert ist resultierend in einer transduzierte Zelllinie getaggt mit der Expressionskassette von V); und wobei die Reihenfolge der Integration der verschiedenen Expressionskassetten nach I, III und V nicht bestimmt ist und wobei die Selektionsmarker i) und ii) unterschiedlich sind zu A oder B.

4. Verfahren zur Generierung einer Master-Producer Zelllinie umfassend die Schritte von
I.) stabiles Integrieren einer Expressionskassette in das Genom einer Wirtszelle oder Zelllinie, diese Expressionskassette umfasst ein Nukleotidkonstrukt umfassend vom 5' zum 3' Ende:
i) eine erste Sequenz, die eine ortsspezifische Integration erlaubt;
ii) ein Promoter;
iii) eine Sequenz, die ein Selektionsmarker A kodiert, diese Sequenz ist operativ mit dem Promoter von ii) verknüpft;
iv) eine zweite Sequenz, die die ortsspezifische Integration ermöglicht, diese Sequenz unterscheidet sich von der ersten Sequenz i);
v) ein nicht-funktionales Selektionsmarker-Fragment Ib, das die Ausbildung eines funktionalen Selektionsmarker I erlaubt wird, wenn es mit dem komplementären Fragment Ia, wie es in der NOI Kassette vorhanden ist, kombiniert wird;
II) Auswahl einer transduzierten Zelllinie worin die Expressionskassette I stabil in das Wirtsgenom integriert ist resultierend in einer transduzierten Zelllinie getaggt mit der Expressionskassette I;
III) stabiles Integrieren einer env Expressionskassette in das Genom dieser Zelle oder Zelllinie, die env Expressionskassette umfasst ein Nukleotidkonstrukt umfassend:
i) gegebenenfalls eine erste Sequenz, die eine ortsspezifische Integration erlaubt;
ii) einen Promoter;
iii) Sequenz(en) für env Gen(e), die operativ mit dem Promoter von ii) verknüpft sind;
iv) gegebenenfalls eine zweite Sequenz, die eine ortsspezifische Integration erlaubt, diese Sequenz unterscheidet sich von der ersten Sequenz aus i);
v) gegebenenfalls ein nicht-funktionale Selektionsmarker-Fragment IIb, das die Ausbildung eines funktionalen Selektionsmarks II erlaubt, wenn es mit dem komplementären Fragment IIa, das sich in der env Expressionskassette befindet, kombiniert wird, dieses Fragment kann gleich oder verschieden sein zu dem Selektionsmarker-Fragment Ib; wobei die Sequenzen, die die ortsspezifische Integration erlauben, gleich oder verschieden sein können zu den Sequenzen in der Expressionskassette nach I.);
IV) Auswahl einer transduzierten Zelllinie, in die die Expressionskassette III stabil in das Wirtsgenom integriert ist, resultierend in eine transduzierte Zelllinie getaggt mit der Expressionskassette von (II.);
V) stabiles Integrieren einer weiteren Expressionskassette in das Genom der Zelle oder Zelllinie, diese Expressionskassette umfasst ein Nukleotidkonstrukt enthaltend ein Promoter, der operativ mit einer Sequenz kodierend gag/pol verknüpft ist, enthält;
VI) Auswahl einer transduzierten Zelllinie, wobei die Expressionskassette von V) stabil in das Wirtsgenom integriert ist resultierend in einer transduzierte Zelllinie getaggt mit der Expressionskassette von V); und wobei die Reihenfolge der Integration der verschiedenen Expressionskassetten nach I, III und V nicht bestimmt ist und wobei die Selektionsmarker i) und ii) unterschiedlich sind zu A oder B.

5. Verfahren nach einem der Ansprüche 1 bis 4, wobei die Expressionskassetten von (I) und/oder (III) weiterhin eine 5' und 3' LTR aufweisen.

6. Verfahren nach einem der Ansprüche 1 bis 5, wobei das Einbringen des Nukleotidkonstrukts/der Nukleotidkonstrukte durch Transfektion oder Infektion durchgeführt wird.

7. Verfahren nach einem der Ansprüche 1, 2, 5, 6, wobei die Integration der Kassetten durch ein Rekombinase-System und/oder durch Restriktionsenzyme erfolgt.

8. Verfahren nach Anspruch 7, wobei das Rekombinase-System das Cre recombinase /loxP Erkennungsstellen von Bakteriophagen P1 oder die ortsspezifische FLP Rekombinase von S. cerevisiae ist.

9. Verfahren nach Anspruch 1 oder 2, wobei das Rekombinationssystem zur ortsspezifischen Integration das FLP System ist unter Verwendung verschiedener FLP Erkennungsziele (FLP recognition target, FRT)-Varianten, wobei die Sequenzen die NOI Kassetten und die env-Kassette auf beiden Seiten flankieren und die die ortsspezifische Rekombination ermöglichen, nicht interagierende Sequenzen, die sich voneinander unterscheiden, sind.

10. Verfahren nach einem der Ansprüche 1, 2, 5 bis 9, wobei das nicht-funktionale Selektionsmarker-Fragment, das sich in der NOI Kassette und/oder der env-Kassette befindet einen Promoter oder ein IRES Element enthält, dieses ist operativ mit einem Startcodon Sequenz verbunden, die die Expression eines Selektionsmarkers erlaubt, der sich von dem Selektionsmarker A oder B unterscheidet, wenn er mit dem komplementären nicht-funktionalen Selektionsmarker-Fragment komplimentiert wird.

11. Verfahren nach einem der Ansprüche 1, 2, 5 bis 10, wobei das nicht-funktionale Selektionsmarker-Fragment, das in der NOI Kassette und/oder der env-Kassette vorhanden ist, nur einen Promoter enthält, der ein stillen aber funktionalen Selektionsmarker, der im Tagging-Schritt integriert wurde, aktiviert.

12. Verfahren nach einem der Ansprüche 1, 2, 5 bis 11 wobei das in der NOI Kassette und/oder env-Kassette vorhandene funktionale Selektionsmarker-Fragment ein Splicesignal aufweist, das die Translation des Selektionsmarkers erlaubt.

13. Verfahren nach Anspruch 1 oder 2, wobei das NOI ein Molekül ausgewählt aus der Gruppe bestehend aus Proteinen, Peptiden, Antisense-Nukleinsäuresequenz, Ribozymen und siRNA kodiert.

14. Verfahren nach Anspruch 1, 2 oder 13, wobei das NOI ein therapeutisch nützliches Molekül ist.

15. Verfahren nach einem der Ansprüche 1, 3, 5 bis 14, wobei die Selektionsmarker A und/oder B ausgewählt sind aus der Gruppe bestehend aus Antibiotika, Wirkstoffresistenzien, zellulären und extrazellulären Expressionsmarkern.

16. Verfahren nach Anspruch 15, wobei die Selektionsmarker A und/oder B ausgewählt sind aus der Gruppe bestehend aus GFP, β-Galactosidase, sekretierter alkalischer Phosphatase und Zelloberflächenmarkern.

17. Verfahren nach einem der Ansprüche 1, 2, 5 bis 16, wobei der Promoter mit dem das NOI und/oder das env-Gen operativ gemäß a.) i) oder b.) i) verknüpft sind, ein LTR ist, das sich stromaufwärts des NOI und/oder env-Gens befindet.

18. Verfahren nach einem der vorherigen Ansprüche, wobei mindestens eine Kassette einen weiteren Promoter aufweist, der operativ mit der zu transkribierenden Sequenz verknüpft ist.

19. Verfahren nach Anspruch 18, wobei der weitere Promoter ausgewählt aus den Gruppen bestehend aus konstitutiven Promotern und Promotoren, die eine zeitlich begrenzte und kontrollierte Expression erlauben.

20. Verfahren nach einem der Ansprüche 1, 2, 5 bis 19, wobei die Rekombinase in die Verpackungszelle durch Transfektion oder Infektion eingebracht wird.

21. Verfahren nach einem der Ansprüche 1, 2, 5 bis 19, wobei die Rekombinase in das System durch direktes Bereitstellen des Enzyms zu den Zellen eingebracht wird.

22. Verfahren nach einem der Ansprüche 1, 2, 5 bis 19, wobei das die Rekombinase kodierende Gen stabil in das Wirtsgenom integriert ist, operativ verknüpft mit einem induzierbaren Promoter.

23. Verfahren nach einem der vorherigen Ansprüche, wobei das Virus pseudotypisiert ist.

24. Virus-produzierende Zelllinie erhältlich mit einem Verfahren nach einem der Ansprüche 1, 2 und 5 bis 23.

25. Master-Producer Zelllinie erhältlich mit einem Verfahren nach einem der Ansprüche 3 bis 22.

26. Verwendung der Virus-produzierenden Zelllinien nach Anspruch 24 oder Master-Producer Zelllinien nach Anspruch 25 zur Herstellung von Viren.

27. Verfahren nach Anspruch 26 zur Herstellung eines therapeutischen Präparats enthaltend ein Virus oder Viruspartikel für die Gentherapie.

## Revendications

1. Procédé de production d'une lignée cellulaire productrice de virus, comprenant les étapes suivantes :
a) introduction, dans une lignée cellulaire productrice mère, d'une construction nucléotidique comprenant une cassette de nucléotides d'intérêt (NOI), comprenant :
i) au moins un NOI lié d'une manière opérationnelle à un promoteur ;
ii) des répétitions terminales longues (LTR) situées respectivement en amont et en aval de la séquence NOI ;
iii) un fragment marqueur de sélection non fonctionnel Ia, situé en aval de la 3'-LTR, permettant la formation d'un marqueur de sélection fonctionnel 1 quand il est combiné à un fragment marqueur complémentaire Ib présent dans la lignée cellulaire productrice mère ; et
iv) des séquences permettant une intégration spécifique de site de la cassette NOI dans une première cassette d'expression qui est présente dans ladite lignée cellulaire productrice mère, lesdites séquences flanquant la cassette NOI sur les deux sites de ladite cassette ; et
b) introduction, dans la lignée cellulaire productrice mère, d'une construction nucléotidique comprenant une cassette d'expression env comprenant :
i) une ou plusieurs séquences d'un ou plusieurs gènes env liées d'une manière opérationnelle à un promoteur ;
ii) un fragment marqueur de sélection non fonctionnel IIa, permettant la formation d'un marqueur de sélection fonctionnel II, qui peut être identique au marqueur de sélection fonctionnel I ou en être différent, quand il est combiné au fragment marqueur complémentaire IIb qui est présent dans la lignée cellulaire productrice mère, ledit fragment marqueur de sélection non fonctionnel IIa étant situé en aval de la ou des séquences du ou des gènes env ;
iii) des séquences permettant une intégration spécifique de site de ladite cassette env dans une deuxième cassette d'expression étiquetée, qui est présente dans ladite lignée cellulaire productrice mère, lesdites séquences flanquant la cassette env sur les deux sites de ladite cassette ;
c) l'initiation du remplacement de la cassette NOI et/ou de la cassette env par la cassette d'expression correspondante qui est présente dans la lignée cellulaire productrice mère ;
d) la sélection des lignées cellulaires dans lesquelles ont été intégrées d'une manière stable la cassette NOI et/ou la cassette env, sur la base du ou des marqueurs de sélection fonctionnels ;
où l'ordre des étapes a) et b) n'est pas déterminé, et les étapes c) et d) peuvent être mises en oeuvre après chacune des étapes a) et b), ou après mise en oeuvre des étapes a) et b) ;
à la condition que la lignée cellulaire productrice mère utilisée respectivement dans a) et b) puisse être obtenue par
I) intégration d'une manière stable d'une cassette d'expression dans le génome d'une cellule ou d'une lignée cellulaire hôte, ladite cassette d'expression comprenant une construction nucléotidique comprenant, de l'extrémité 5' à l'extrémité 3' :
i) une première séquence permettant une intégration spécifique de site ;
ii) un promoteur ;
iii) une séquence codant pour un marqueur de sélection A, ladite séquence étant liée d'une manière opérationnelle au promoteur de ii) ;
iv) une deuxième séquence permettant une intégration spécifique de site, ladite séquence étant différente de la première séquence de i) ;
v) un fragment marqueur de sélection non fonctionnel Ib, permettant la formation d'un marqueur de sélection fonctionnel I quand il est combiné au fragment complémentaire la qui est présent dans la cassette NOI ;
II) sélection d'une lignée cellulaire transduite, dans laquelle la cassette d'expression de (I) est intégrée d'une manière stable dans le génome hôte, en conduisant à une lignée cellulaire transduite qui est étiquetée par la cassette d'expression de I) ;
III) intégration d'une manière stable d'une cassette d'expression différente dans le génome de la cellule ou de la lignée cellulaire, ladite cassette d'expression comprenant une construction nucléotidique comprenant, de l'extrémité 5' à l'extrémité 3' :
i) une première séquence permettant une intégration spécifique de site ;
ii) un promoteur ;
iii) une séquence codant pour un marqueur de sélection B, qui est différent du marqueur de sélection A, ladite séquence étant liée d'une manière opérationnelle au promoteur de ii) ;
iv) une deuxième séquence permettant une intégration spécifique de site, ladite séquence étant différente de la première séquence de i);
v) un fragment marqueur de sélection non fonctionnel IIb, permettant la formation d'un marqueur de sélection fonctionnel II quand il est combiné au fragment complémentaire IIa qui est présent dans la cassette d'expression env, qui peut être identique au fragment marqueur de sélection Ib, ou en être différent ;
où les séquences permettant une intégration spécifique de site peuvent être identiques auxdites séquences de la cassette d'expression de I), ou en être différentes ;
IV) sélection d'une lignée cellulaire transduite, dans laquelle la cassette d'expression de (III) est intégrée d'une manière stable dans le génome hôte, en conduisant à une lignée cellulaire transduite qui est étiquetée par la cassette d'expression de (III) ;
V) intégration d'une manière stable d'une cassette d'expression différente dans le génome de la cellule ou de la lignée cellulaire, ladite cassette d'expression comprenant une construction nucléotidique contenant un promoteur lié d'une manière opérationnelle à une séquence codant pour gag/pol ;
VI) sélection d'une lignée cellulaire transduite, dans laquelle la cassette d'expression de (V) est intégrée d'une manière stable dans le génome hôte, en conduisant à une lignée cellulaire transduite, étiquetée par la cassette d'expression de V ;
et où l'ordre d'intégration des différentes cassettes d'expression selon (I), (III) et (V) n'est pas déterminé, les marqueurs de sélection I et II étant différents de A ou B.

2. Procédé de production d'une lignée cellulaire productrice de virus, comprenant les étapes suivantes :
a) introduction, dans une lignée cellulaire productrice mère, d'une construction nucléotidique comprenant une cassette de nucléotides d'intérêt (NOI) comprenant :
i) au moins un NOI lié d'une manière opérationnelle à un promoteur ;
ii) des répétitions terminales longues LTR situées respectivement en amont et en aval de la séquence NOI ;
iii) un fragment de sélection non fonctionnel Ia, situé en aval de la 3'-LTR, permettant la formation d'un domaine de sélection fonctionnel I quand il est combiné au fragment complémentaire Ib qui est présent dans la lignée cellulaire productrice mère ; et
iv) des séquences permettant une intégration spécifique de site de la cassette NOI dans une première cassette d'expression qui est présente dans ladite lignée cellulaire productrice mère, lesdites séquences flanquant la cassette NOI sur les deux sites de ladite cassette ; et, en option
b) introduction, dans la lignée cellulaire productrice mère, d'une construction nucléotidique comprenant une cassette d'expression env, comprenant
i) une ou plusieurs séquences d'un ou plusieurs gènes env liées d'une manière opérationnelle à un promoteur ;
ii) un fragment marqueur de sélection non fonctionnel IIa, permettant la formation d'un marqueur de sélection fonctionnel II, qui peut être identique au marqueur de sélection fonctionnel I ou en être différent, quand il est combiné au fragment marqueur complémentaire IIb qui est présent dans la lignée cellulaire productrice mère, ledit fragment marqueur de sélection non fonctionnel IIa étant situé en aval de la ou des séquences du ou des gènes env ;
iii) des séquences permettant une intégration spécifique de site de ladite cassette env dans une cassette d'expression env qui est présente dans ladite lignée cellulaire productrice mère, lesdites séquences flanquant la cassette env sur les deux sites de ladite cassette ;
c) initiation du remplacement de la cassette NOI et/ou de la cassette env par la cassette d'expression correspondante, qui est présente dans la lignée cellulaire productrice mère ;
d) sélection des lignées cellulaires dans lesquelles la cassette NOI et/ou la cassette env ont été intégrées d'une manière stable, sur la base du ou des domaines de sélection fonctionnels ;
où l'ordre des étapes a) et en option b) n'est pas déterminé, les étapes c) et d) pouvant être mises en oeuvre après chacune des étapes a) et b), ou après mise en oeuvre des étapes a) et b) ;
à la condition que la lignée cellulaire productrice mère utilisée respectivement en a) et b) puisse être obtenue par
I) intégration d'une manière stable d'une cassette d'expression dans le génome d'une cellule ou d'une lignée cellulaire hôte, ladite cassette d'expression comprenant une construction nucléotidique comprenant, de l'extrémité 3' à l'extrémité 5' :
i) une première séquence permettant une intégration spécifique de site ;
ii) un promoteur ;
iii) une séquence codant pour un marqueur de sélection A, ladite séquence étant liée d'une manière opérationnelle au promoteur de ii) ;
iv) une deuxième séquence permettant une intégration spécifique de site, ladite séquence étant différente de la première séquence de i) ;
v) un fragment marqueur de sélection non fonctionnel Ib, permettant la formation d'un marqueur de sélection fonctionnel 1 quand il est combiné au fragment complémentaire Ia qui est présent dans la cassette NOI ;
II) sélection d'une lignée cellulaire transduite, dans laquelle la cassette d'expression de (I) est intégrée d'une manière stable dans le génome hôte, conduisant à une lignée cellulaire transduite étiquetée par la cassette d'expression de I) ;
III) intégration d'une manière stable d'une cassette d'expression env dans le génome de ladite cellule ou de ladite lignée cellulaire, ladite cassette d'expression env comprenant une construction nucléotidique comprenant :
i) en option, une première séquence permettant une intégration spécifique de site ;
ii) un promoteur ;
iii) une ou plusieurs séquences d'un ou plusieurs gènes env liées d'une manière opérationnelle au promoteur de ii) ;
iv) en option, une deuxième séquence permettant une intégration spécifique de site, ladite séquence étant différente de la première séquence de i) ;
v) en option, un fragment marqueur de sélection non fonctionnel IIb, permettant la formation d'un marqueur de sélection fonctionnel II quand il est combiné au fragment complémentaire IIa qui est présent dans la cassette d'expression env, et qui peut être identique au fragment marqueur de sélection Ib ou en être différent ;
où les séquences permettant une intégration spécifique de site peuvent être identiques auxdites séquences de la cassette d'expression de I), ou en être différentes ;
(IV) sélection d'une lignée cellulaire transduite, dans laquelle la cassette d'expression de (III) est intégrée d'une manière stable dans le génome hôte, conduisant à une lignée cellulaire transduite étiquetée par la cassette d'expression de (III) ;
(V) intégration d'une manière stable d'une cassette d'expression différente dans le génome de la cellule ou de la lignée cellulaire, ladite cassette d'expression comprenant une construction nucléotidique contenant un promoteur lié d'une manière opérationnelle à une séquence codant pour gag/pol ;
(VI) sélection d'une lignée cellulaire transduite, dans laquelle la cassette d'expression de (V) est intégrée d'une manière stable dans le génome hôte, conduisant à une lignée cellulaire transduite étiquetée par la cassette d'expression de V ;
et où l'ordre d'intégration des différentes cassettes d'expression selon (I), (III) et (V), n'est pas déterminé, les marqueurs de sélection I et II étant différents du marqueur de sélection A ou B.

3. Procédé de production d'une lignée cellulaire productrice mère, comprenant les étapes de :
I) intégration d'une manière stable d'une cassette d'expression dans le génome d'une cellule hôte ou d'une lignée cellulaire, ladite cassette d'expression comprenant une construction nucléotidique comprenant, de l'extrémité 5' à l'extrémité 3' :
i) une première séquence permettant une intégration spécifique de site ;
ii) un promoteur ;
iii) une séquence codant pour un marqueur de sélection A, ladite séquence étant liée d'une manière opérationnelle au promoteur de ii) ;
iv) une deuxième séquence permettant une intégration spécifique de site, ladite séquence étant différente de la première séquence de i) ;
v) un fragment marqueur de sélection non fonctionnel Ib, permettant la formation d'un marqueur de sélection fonctionnel I quand il est combiné au fragment complémentaire Ia qui est présent dans la cassette NOI ;
II) sélection d'une lignée cellulaire transduite, dans laquelle la cassette d'expression de (I) est intégrée d'une manière stable dans le génome hôte, conduisant à une lignée cellulaire transduite étiquetée par la cassette d'expression de I) ;
III) intégration d'une manière stable d'une cassette d'expression différente dans le génome de la cellule ou de la lignée cellulaire, ladite cassette d'expression comprenant une construction nucléotidique comprenant, de l'extrémité 5' à l'extrémité 3' :
i) une première séquence permettant une intégration spécifique de site ;
ii) un promoteur ;
iii) une séquence codant pour un marqueur de sélection B, qui est différent du marqueur de sélection A, ladite séquence étant liée d'une manière opérationnelle au promoteur de ii) ;
iv) une deuxième séquence permettant une intégration spécifique de site, ladite séquence étant différente de la première séquence de i) ;
v) un fragment marqueur de sélection non fonctionnel IIb, permettant la formation d'un marqueur de sélection fonctionnel II quand il est combiné au fragment complémentaire IIa qui est présent dans la cassette d'expression env, qui peut être identique au fragment marqueur de sélection Ib, ou en être différent ;
où les séquences permettant une intégration spécifique de site peuvent être identiques auxdites séquences de la cassette d'expression de I), ou en être différentes ;
IV) sélection d'une lignée cellulaire transduite, dans laquelle la cassette d'expression de (III) est intégrée d'une manière stable dans le génome hôte, conduisant à une lignée cellulaire transduite étiquetée par la cassette d'expression de (III) ;
V) intégration d'une manière stable d'une cassette d'expression différente dans le génome de la cellule ou de la lignée cellulaire, ladite cassette d'expression comprenant une construction nucléotidique contenant un promoteur lié d'une manière opérationnelle à une séquence codant pour gag/pol ;
VI) sélection d'une lignée cellulaire transduite, dans laquelle la cassette d'expression de (V) est intégrée d'une manière stable dans le génome hôte, conduisant à une lignée cellulaire transduite étiquetée par la cassette d'expression de V ;
et où l'ordre d'intégration des différentes cassettes d'expression selon (I), (III) et (V) n'est pas déterminé.

4. Procédé de production d'une lignée cellulaire productrice mère, comprenant les étapes de :
I) intégration d'une manière stable d'une cassette d'expression dans le génome d'une cellule ou d'une lignée cellulaire hôte, ladite cassette d'expression comprenant une construction nucléotidique comprenant, de l'extrémité 3' à l'extrémité 5' :
i) une première séquence permettant une intégration spécifique de site ;
ii) un promoteur ;
iii) une séquence codant pour un marqueur de sélection A, ladite séquence étant liée d'une manière opérationnelle au promoteur de ii) ;
iv) une deuxième séquence permettant une intégration spécifique de site, ladite séquence étant différente de la première séquence de i) ;
v) un fragment marqueur de sélection non fonctionnel Ib, permettant la formation d'un marqueur de sélection fonctionnel I quand il est combiné au fragment complémentaire Ia qui est présent dans la cassette NOI ;
II) sélection d'une lignée cellulaire transduite, dans laquelle la cassette d'expression de (I) est intégrée d'une manière stable dans le génome hôte, conduisant à une lignée cellulaire transduite étiquetée par la cassette d'expression de I) ;
III) intégration d'une manière stable d'une cassette d'expression env dans le génome de ladite cellule ou de ladite lignée cellulaire, ladite cassette d'expression comprenant une construction nucléotidique comprenant :
i) en option, une première séquence permettant une intégration spécifique de site ;
ii) un promoteur ;
iii) une ou plusieurs séquences d'un ou plusieurs gènes env liées d'une manière opérationnelle au promoteur de ii) ;
iv) en option, une deuxième séquence permettant une intégration spécifique de site, ladite séquence étant différente de la première séquence de i) ;
v) en option, un fragment marqueur de sélection non fonctionnel IIb, permettant la formation d'un marqueur de sélection fonctionnel II quand il est combiné au fragment complémentaire IIa dans la cassette d'expression env, qui peut être identique au fragment marqueur de sélection Ib ou en être différent ;
où les séquences permettant une intégration spécifique de site peuvent être identiques auxdites séquences de la cassette d'expression de I), ou en être différentes ;
(IV) sélection d'une lignée cellulaire transduite, dans laquelle la cassette d'expression de (III) est intégrée d'une manière stable dans le génome hôte, conduisant à une lignée cellulaire transduite étiquetée par la cassette d'expression de (III) ;
(V) intégration d'une manière stable d'une cassette d'expression différente dans le génome de la cellule ou de la lignée cellulaire, ladite cassette d'expression comprenant une construction nucléotidique contenant un promoteur lié d'une manière opérationnelle à une séquence codant pour gag/pol ;
(VI) sélection d'une lignée cellulaire transduite, dans laquelle la cassette d'expression de (V) est intégrée d'une manière stable dans le génome hôte, conduisant à une lignée cellulaire transduite étiquetée par la cassette d'expression de V ;
et où l'ordre d'intégration des différentes cassettes d'expression selon (I), (III) et (V) n'est pas déterminé.

5. Procédé selon l'une quelconque des revendications 1 à 4, dans lequel les cassettes d'expression de (I) et/ou (III) contiennent en outre une 5'- et une 3'-LTR.

6. Procédé selon l'une quelconque des revendications 1 à 5, dans lequel l'introduction de la ou des constructions nucléotidiques est réalisée par transfection ou par une infection.

7. Procédé selon l'une quelconque des revendications 1, 2, 5, 6, dans lequel l'intégration des cassettes est réalisée par un système de recombinases et/ou par des enzymes de restriction.

8. Procédé selon la revendication 7, dans lequel le système de recombinases est constitué des sites de reconnaissance Cre recombinase/loxP du bactériophage P1, ou la FLP recombinase spécifique de site de S. cerevisiae.

9. Procédé selon la revendication 1 ou 2, dans lequel le système de recombinaison, pour une intégration spécifique de site, est le système FLP, utilisant différents variants de la cible de reconnaissance du FLP (FRT), où les séquences flanquant la cassette NOI et la cassette env sur les deux sites, et qui permettent une recombinaison spécifique de site, sont des séquences non interagissantes, différentes l'une de l'autre.

10. Procédé selon l'une quelconque des revendications 1, 2, 5 à 9, dans lequel le fragment marqueur de sélection non fonctionnel qui est présent dans la cassette NOI et/ou la cassette env contient un promoteur ou un élément IRES lié d'une manière opérationnelle à une séquence de codons d'initiation, permettant l'expression d'un marqueur de sélection différent du marqueur de sélection A ou B quand il est complémenté par le fragment marqueur de sélection non fonctionnel complémentaire.

11. Procédé selon l'une quelconque des revendications 1, 2, 5 à 10, dans lequel le fragment marqueur de sélection non fonctionnel qui est présent dans la cassette NOI et/ou la cassette env ne contient qu'un promoteur qui active un marqueur de sélection, silencieux mais fonctionnel, intégré lors de l'étape d'étiquetage.

12. Procédé selon l'une quelconque des revendications 1, 2, 5 à 11, dans lequel le fragment marqueur de sélection non fonctionnel qui est présent dans la cassette NOI et/ou la cassette env contient un signal d'épissage qui permet la traduction du marqueur de sélection.

13. Procédé selon la revendication 1 ou 2, dans lequel le NOI code pour une molécule choisie dans le groupe consistant en les protéines, les peptides, les séquences d'acides nucléiques antisens, les ribozymes et l'ARNsi.

14. Procédé selon la revendication 1, 2 ou 13, dans lequel le NOI est une molécule thérapeutiquement utile.

15. Procédé selon l'une quelconque des revendications. 1, 3, 5 à 14, dans lequel les marqueurs de sélection A et/ou B sont choisis dans le groupe consistant en les marqueurs de résistance aux antibiotiques, de résistance aux médicaments, d'expression cellulaire et extracellulaire.

16. Procédé selon la revendication 15, dans lequel les marqueurs de sélection A et/ou B sont choisis dans le groupe consistant en les marqueurs de GFP, de β-galactosidase, de phosphatase alcaline sécrétée et de surface cellulaire.

17. Procédé selon l'une quelconque des revendications 1, 2, 5 à 16, dans lequel le promoteur auquel le NOI et/ou le gène env est lié d'une manière opérationnelle selon a)i) ou b)i) est une LTR située en amont, respectivement du NOI et/ou du gène env.

18. Procédé selon l'une quelconque des revendications précédentes, dans lequel au moins une cassette contient un promoteur additionnel lié d'une manière opérationnelle aux séquences à transcrire.

19. Procédé selon la revendication 18, dans lequel le promoteur additionnel est choisi dans le groupe consistant en les promoteurs constitutifs et les promoteurs pour une expression restreinte dans le temps ou contrôlée.

20. Procédé selon l'une quelconque des revendications 1, 2, 5 à 19, dans lequel la recombinase est introduite dans la cellule d'empaquetage par transfection ou infection.

21. Procédé selon l'une quelconque des revendications 1, 2, 5 à 19, dans lequel la recombinase est introduite dans le système par amenée directe de l'enzyme aux cellules.

22. Procédé selon l'une quelconque des revendications 1, 2, 5 à 19, dans lequel le gène codant pour la recombinase est intégré d'une manière stable dans le génome hôte lié d'une manière opérationnelle à un promoteur inductible.

23. Procédé selon l'une quelconque des revendications précédentes, dans lequel le virus est pseudotypé.

24. Lignée cellulaire productrice de virus pouvant être obtenue par un procédé selon l'une quelconque des revendications 1, 2 et 5 à 23.

25. Lignée cellulaire productrice mère pouvant être obtenue par un procédé selon l'une quelconque des revendications 3 à 22.

26. Utilisation de la lignée cellulaire productrice de virus selon la revendication 24 ou de la lignée cellulaire productrice mère selon la revendication 25 pour la production d'un virus.

27. Utilisation selon la revendication 26, pour la production d'une préparation thérapeutique contenant un virus ou une particule virale pour une thérapie génique.
